## Europäisches Patentamt
## European Patent Office
## Office européen des brevets

(19)

(11) **EP 1 118 621 A1**

(12) **EUROPEAN PATENT APPLICATION**
published in accordance with Art. 158(3) EPC

(43) Date of publication:
**25.07.2001 Bulletin 2001/30**

(51) Int Cl.⁷: **C07K 14/705**, C07K 16/28,
C12N 1/21, C12N 15/12,
C12P 21/02, G01N 33/68,
A61K 38/00

(21) Application number: **99969941.6**

(22) Date of filing: **30.09.1999**

(86) International application number:
**PCT/JP99/05365**

(87) International publication number:
**WO 00/20456 (13.04.2000 Gazette 2000/15)**

(84) Designated Contracting States:
**AT BE CH CY DE DK ES FI FR GB GR IE IT LI LU
MC NL PT SE**
Designated Extension States:
**AL LT LV MK RO SI**

(30) Priority: **01.10.1998 JP 27951298
20.10.1998 JP 29866798**

(71) Applicant: **Takeda Chemical Industries, Ltd.
Osaka-shi, Osaka 541-0045 (JP)**

(72) Inventors:
• **WATANABE, Takuya
Tsukuba-shi, Ibaraki 305-0821 (JP)**
• **TERAO, Yasuko
Tsukuba-shi, Ibaraki 305-0034 (JP)**
• **FUKUSUMI, Shoji
Tsukuba-shi, Ibaraki 305-0044 (JP)**

(74) Representative: **Keller, Günter, Dr. et al
Lederer, Keller & Riederer
Patentanwälte
Prinzregentenstrasse 16
80538 München (DE)**

(54) **NOVEL G PROTEIN-COUPLED RECEPTOR PROTEIN AND DNA THEREOF**

(57) The present invention relates to a human-derived G-protein coupled receptor protein or a partial peptide thereof or a salt thereof, a nucleic acid encoding said receptor protein and a derivative thereof.

A G-protein coupled receptor protein derived from a human hippocampus according to the invention or a nucleic acid encoding said protein and a derivative thereof can be used in determining a ligand (agonist) for a G-protein coupled receptor protein of the invention, in a preventing and/or treating agent against a disease related to a dysfunction of a G-protein coupled receptor protein of the invention, in a gene diagnostic agent, and in screening for a compound capable of altering the expression level of a receptor protein of the invention or a partial peptide thereof.

**EP 1 118 621 A1**

**Description**

<u>Technical Field</u>

The present invention relates to a novel human G-protein coupled receptor protein or a salt thereof and a DNA encoding the same.

<u>Background of the Invention</u>

Many bioactive substances such as hormones and neurotransmitters serve to regulate the biological functions via specific receptor proteins which are present in cell membranes. Since most of such receptor proteins effect an intracellular signal transmission via the activation of a guanine nucleotide-binding protein (hereinafter sometimes referred to as G protein) coupling therewith and have a common structure containing 7 transmembrane regions, they are referred to together as G-protein coupled receptor proteins or 7 transmembrane receptor proteins (7TMR).

A G-protein coupled receptor protein is present on each functional cell surface in an in vivo cell or organ, and plays a physiologically important role as a target of a molecule which regulates the function of such cell or organ, including hormones, neurotransmitters and bioactive substances. A receptor transmits a signal to the inside of a cell via the binding to a bioactive substance, and such signal induces various reactions such as the activation or inhibition of the cell.

To clarify the relationship between a substance which regulates a complicated function in every in vivo cell or organ and a receptor protein specific thereto, especially a G-protein coupled receptor protein, serves to provide a valuable means for understanding the function in a cell or an organ of various organisms and for developing a pharmaceutical related closely to such function.

For example, the physiological function of every in vivo organ is regulated under the control of a diversity of hormones, hormone-like substances, neurotransmitters and bioactive substances. Especially, bioactive substances are present in various in vivo locations, and regulate the physiological functions in such locations via the relevant receptor proteins. Many in vivo hormones, neurotransmitters and other bioactive substances still remain uncharacterized, and their receptor protein structures have scarcely been reported. Moreover, even as to known receptor proteins, it has been poorly be clarified whether its subtype exists or not.

To characterize the relationship between a substance regulating a complicated in vivo function and a receptor protein specific thereto is a very important means in developing a pharmaceutical. In addition, for the purpose of screening efficiently for an agonist and an antagonist for the receptor protein described above and to develop a pharmaceutical, it is essential to understand the function of a gene encoding a receptor protein expressed in vivo and to express the same in a suitable expression system.

Recently, studies on the random analysis of cDNA sequences as a means for analyzing a gene expressed in vivo are being extensively performed, and fragment sequences of such cDNA thus obtained is registered as an Expressed Sequence Tag (EST) in a data base, where it is disclosed. However, most of such ESTs contain only sequence data, from which the relevant function is not evident.

A substance having an inhibitory effect on the binding between a G-protein coupled receptor and a bioactive substance (i.e., ligand) or a substance effecting a signal transmission similarly to a bioactive substance (i.e., ligand) as a result of binding has been utilized as an antagonist or an agonist specific to such receptor in a pharmaceutical which regulates an in vivo function. Accordingly, to identify a novel G-protein coupled receptor protein which is not only important in an in vivo physiological expression but also can serve as a target in developing a pharmaceutical and to clone its gene (for example, cDNA) serves as a very important means for identifying a novel G-protein coupled receptor protein-specific ligand, agonist or antagonist.

Nevertheless, not all G-protein coupled receptors have been identified, and there are currently still many unknown G-protein coupled receptors and so-called orphan receptors whose relevant ligands are not identified, thus causing a demand for exploring a novel G-protein coupled receptor as well as for clarifying the function thereof.

A G-protein coupled receptor is useful in searching for a novel bioactive substance (i.e., ligand) and also in searching for an agonist or an antagonist for such receptor utilizing its signal transmitting effect as an index. On the other hand, an agonist or an antagonist for such receptor can be produced by analyzing the physiological effect of the receptor based on an inactivation experiment of the receptor (in a knockout animal), even if no physiological ligand can be identified. A ligand, an agonist or an antagonist for such receptor is expected to be utilized as a preventing and/ or treating agent or a diagnostic agent for a disease related to the dysfunction of a G-protein coupled receptor.

In addition, a reduced or increased in vivo function of a G-protein coupled receptor resulting from a gene variation of such receptor frequently induces a certain disease. In such a case, gene therapy by introducing the receptor gene into a living body (or into a certain organ) or by introducing an antisense nucleic acid into the receptor gene is possible in addition to the administration of an antagonist or an agonist to the receptor. For this purpose, the base sequence of the receptor is essential information for detecting a deletion or a variation in the gene, and a gene of the receptor is applicable to a preventing and/or treating agent or a diagnostic agent for a disease related to the dysfunction of the receptor.

Disclosure of the Invention

The present invention is intended to provide a novel human G-protein coupled receptor protein which is useful as discussed above. Thus, a novel human G-protein coupled receptor protein or a partial peptide or a salt thereof, a polynucleotide (DNA, RNA or a derivative thereof) comprising a polynucleotide (DNA, RNA or a derivative thereof) encoding such G-protein coupled receptor protein or a partial peptide thereof, a recombinant vector comprising such polynucleotide, a transformant containing such recombinant vector, a method for producing such G-protein coupled receptor protein or a salt thereof, an antibody against such G-protein coupled receptor protein or a partial peptide or a salt thereof, a compound which alters the expression level of such G-protein coupled receptor protein, a method for determining a ligand for such G-protein coupled receptor protein, a method for screening for a compound (antagonist, agonist) or a salt thereof capable of altering the binding affinity between a ligand and such G-protein coupled receptor protein, a kit for such screening, a compound (antagonist, agonist) or a salt there of capable of altering the binding affinity between a ligand and such G-protein coupled receptor protein which can be obtained by using such screening method or screening kit, and, a pharmaceutical comprising a compound (antagonist, agonist) or a salt thereof capable of altering the binding affinity between a ligand and such G-protein coupled receptor protein or a compound capable of altering the expression level of such G-protein coupled receptor protein are provided here.

We made an effort and then discovered that a cDNA encoding a novel G-protein coupled receptor protein derived from a human hippocampus can successfully be isolated and sequenced for its entire bases based on an EST database prepared by a degenerated PCR. Then we found the first to seventh transmembrane regions on a hydrophobic plot based on the translation from the base sequence described above into an amino acid sequence, and ensured that the proteins encoded by these cDNAs were the G-protein coupled receptor proteins of the 7 transmembrane region type. Based on these findings, we made a further effort and finally established the invention.

Thus, the present invention relates to:

(1) a G-protein coupled receptor protein comprising an amino acid sequence identical or substantially identical to the amino acid sequence represented by SEQ ID NO.1 or a salt thereof;

(2) a G-protein coupled receptor protein according to Section (1) described above wherein an amino acid sequence substantially identical to the amino acid sequence represented by SEQ ID NO.1 is the amino acid sequence represented by SEQ ID NO.6 or a salt thereof;

(3) a partial peptide of a G-protein coupled receptor protein according to Section (1) described above or a salt thereof;

(4) a polynucleotide comprising a polynucleotide having a base sequence encoding a G-protein coupled receptor protein according to Section (1) described above;

(5) a polynucleotide according to Section (4) described above which is a DNA;

(6) a polynucleotide according to Section (4) described above having the base sequence represented by SEQ ID NO.2 or SEQ ID NO.3;

(7) a polynucleotide according to Section (4) described above comprising a polynucleotide having a base sequence encoding a G-protein coupled receptor protein comprising the amino. acid sequence represented by SEQ ID NO.6;

(8) a polynucleotide according to Section (7) described above which is a DNA;

(9) a polynucleotide according to Section (7) described above having the base sequence represented by SEQ ID NO.7;

(10) a recombinant vector comprising a polynucleotide according to Section (4) described above;

(11) a transformant transformed with a recombinant vector according to Section (10) described above;

(12) a method for producing a G-protein coupled receptor protein according to Section (1) described above or a salt thereof comprising incubating a transformant according to Section (11) described above to produce a G-protein coupled receptor protein according to Section (1) described above;

(13) an antibody directed to a G-protein coupled receptor protein according to Section (1) described above or a partial peptide according to Section (3) described above or a salt thereof;

(14) an antibody according to Section (13) described above which is a neutralizing antibody which inactivates the signal transmission of a G-protein coupled receptor protein according to Section (1) described above;

(15) a diagnostic agent comprising an antibody according to Section (13) described above;

(16) a ligand for a G-protein coupled receptor protein according to Section (1) described above or a salt thereof obtained by using a G-protein coupled receptor protein according to Section (1) described above or a partial peptide according to Section (3) described above or a salt thereof;

(17) a pharmaceutical comprising a ligand for a G-protein coupled receptor protein according to Section (16) described above;

(18) a method for determining a ligand for a G-protein coupled receptor protein according to Section (1) described above or a salt thereof comprising using a G-protein coupled receptor protein according to Section (1) described above or a partial peptide according to Section (3) described above or a salt thereof;

(19) a method for screening for a compound or a salt thereof which is capable of altering the binding affinity between a ligand and a G-protein coupled receptor protein according to Section (1) described above or a salt thereof comprising using a G-protein coupled receptor protein according to Section (1) described above or a partial peptide according to Section (3) described above or a salt thereof;

(20) a kit for screening for a compound or a salt thereof capable of altering the binding affinity between a ligand and a G-protein coupled receptor protein according to Section (1) described above or a salt thereof comprising using a G-protein coupled receptor protein according to Section (1) described above or a partial peptide according to Section (3) described above or a salt thereof;

(21) a compound or a salt thereof capable of altering the binding affinity between a ligand and a G-protein coupled receptor protein according to Section (1) described above or a salt thereof which can be obtained by using a screening method according to Section (19) described above or a screening kit according to Section (20) described above;

(22) a pharmaceutical comprising a compound or a salt thereof capable of altering the binding affinity between a ligand and a G-protein coupled receptor protein according to Section (1) described above or a salt thereof which can be obtained by using a screening method according to Section (19) described above or a screening kit according to Section (20) described above;

(23) a polynucleotide capable of being hybridized with a polynucleotide according to Section (4) described above under a high stringent condition;

(24) a polynucleotide comprising a base sequence or a part thereof which is complementary with a polynucleotide according to Section (4) described above;

(25) a method for quantifying an mRNA of a G-protein coupled receptor protein according to Section (1) described above comprising using a polynucloeotide according to Section (4) described above or a part thereof;

(26) a method for quantifying a G-protein coupled receptor protein according to Section (1) described above comprising using an antibody according to Section (13) described above;

(27) a method for diagnosing a disease related to a function of a G-protein coupled receptor protein according to Section (1) described above comprising using a quantification method according to Section (25) described above or Section (26) described above;

(28) a method for screening for a compound or a salt thereof capable of altering the expression level of a G-protein coupled receptor protein according to Section (1) described above comprising using a quantification method according to Section (25) described above;

(29) a method for screening for a compound or a salt thereof capable of altering the amount of a G-protein coupled receptor protein according to Section (1) described above in a cell membrane comprising using a quantification method according to Section (26) described above;

(30) a compound or a salt thereof capable of altering the expression level of a G-protein coupled receptor protein according to Section (1) described above which can be obtained by using a screening method according to Section (28) described above; and,

(31) a compound or a salt thereof capable of altering the amount of a G-protein coupled receptor protein according to Section (1) described above in a cell membrane which can be obtained by using a screening method according to Section (29) described above.

Those also provided are:

(32) a G-protein coupled receptor protein according to Section (1) described above or a salt thereof wherein said protein is a protein comprising [1] an amino acid sequence formed as a result of the deletion of one or more (preferably 1 to about 30, more preferably 1 to about 9, most preferably 1 to 5) amino acids in the amino acid sequence represented by Sequence ID. NO.1, [2] an amino acid sequence formed as a result of the addition of one or more (preferably 1 to about 30, more preferably 1 to about 10, most preferably 1 to 5) amino acids to the amino acid sequence represented by Sequence ID. NO.1, [3] an amino acid sequence formed as a result of the substitution of one or more (preferably 1 to about 30, more preferably 1 to about 10, most preferably 1 to 5) amino acids in the amino acid sequence represented by Sequence ID. NO.1 by other amino acids or [4] an amino acid as a combination thereof;

(33) a method for determining a ligand according to Section (18) described above comprising bringing a G-protein coupled receptor protein according to Section (1) described above or a salt thereof or a partial peptide according to Section (3) described above or a salt thereof into contact with a test substance;

(34) a method for determining a ligand according to Section (33) wherein said ligand is, for example, angiotensin, bonbesin, cannabinoid, cholecystokinin, glutamin, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal polypeptide), somatostatin, dopamine, motilin, amylin, bradykinin, CGRP (calcitonin gene-related peptide), leucotriene, pancreastatin, prostaglandine, thromboxane, adenosine, adrenaline, α and β-chemokine (for example, IL-8, GROα, GROβ, GROγ, NAP-2, ENA-78, PF4, IP10, GCP-2, MCP-1, HC14, MCP-3, I-309, NIP1α, MIP-1β, RANTES and the like), endoserine, enterogastrin, histamine, neurotensin, TRH, pancreatic polypeptide or galanin;

(35) a screening method according to Section (19) described above comprising a comparison between (i) a contact of a G-protein coupled receptor protein according to Section (1) described above or a salt thereof or a partial peptide according to Section (3) described above or a salt thereof with a ligand and (ii) a contact of the G-protein coupled receptor protein according to Section (1) described above or the salt thereof or the partial peptide according to Section (3) described above or the salt thereof with the ligand and a test compound;

(36) a method for screening a compound or a salt thereof capable of altering the binding affinity between a ligand and a G-protein coupled receptor protein according to Section (1) described above or a salt thereof comprising a determination of and a comparison between the levels of the binding of a labeled ligand to the G-protein coupled receptor protein according to Section (1) described above or the salt thereof or a partial peptide according to Section (3) described above or a salt thereof upon (i) a contact of the labeled ligand with the G-protein coupled receptor protein according to Section (1) described above or the salt thereof or the partial peptide according to Section (3) described above or the salt thereof and upon (ii) a contact of the labeled ligand and a test compound with the G-protein coupled receptor protein according to Section (1) described above or the salt thereof or the partial peptide according to Section (3) described above or the salt thereof;

(37) a method for screening a compound or a salt thereof capable of altering the binding affinity between a ligand and a G-protein coupled receptor protein according to Section (1) described above or a salt thereof comprising a determination of and a comparison between the levels of the binding of a labeled ligand to a cell comprising the G-protein coupled receptor protein according to Section (1) described above upon (i) a contact of the labeled ligand with said cell and upon (ii) a contact of the labeled ligand and a test substance with said cell;

(38) a method for screening a compound or a salt thereof capable of altering the binding affinity between a ligand and a G-protein coupled receptor protein according to Section (1) described above or a salt thereof comprising a determination of and a comparison between the levels of the binding of a labeled ligand to a membrane fraction of a cell comprising a G-protein coupled receptor protein according to Section (1) described above upon (i) a contact of the labeled ligand with the membrane fraction of said cell and upon (ii) a contact of the labeled ligand and a test substance with the membrane fraction of said cell;

(39) a method for screening a compound or a salt thereof capable of altering the binding affinity between a ligand and a G-protein coupled receptor protein according to Section (1) described above or a salt thereof expressed on the cell membrane of a transformant according to Section (11) described above by incubating said transformant comprising a determination of and a comparison between the levels of the binding of a labeled ligand to said G-protein coupled receptor protein upon (i) a contact of the labeled ligand with said G-protein coupled receptor protein and upon (ii) a contact of the labeled ligand and a test substance with said G-protein coupled receptor protein;

(40) a method for screening a compound or a salt thereof capable of altering the binding affinity between a ligand and a G-protein coupled receptor protein according to Section (1) described above or a salt thereof comprising a determination of and a comparison between the cell stimulating activities via the G-protein coupled receptor protein upon (i) a contact of a compound activating the G-protein coupled receptor protein according to Section (1) described above or a salt thereof with a cell comprising the G-protein coupled receptor protein according to Section (1) described above and upon (ii) a contact of a compound activating the G-protein coupled receptor protein according to Section (1) described above or a salt thereof and a test compound with a cell comprising the G-protein coupled receptor protein according to Section (1) described above;

(41) a method for screening a compound or a salt thereof capable of altering the binding affinity between a ligand and a G-protein coupled receptor protein according to Section (1) described above or a salt thereof comprising a determination of and a comparison between the cell stimulating activities via the G-protein coupled receptor protein upon a contact of a compound activating the G-protein coupled receptor protein according to Section (1) described above or a salt thereof with the G-protein coupled receptor protein expressed on the cell membrane of a transformant according to Section (11) described above by incubating said transformant and upon a contact of a compound activating the G-protein coupled receptor protein according to Section (1) described above or a salt thereof and a test compound with the G-protein coupled receptor protein expressed on the cell membrane of the transformant according to Section (11) described above by incubating said transformant;

(42) a screening method according to Section (40) or (41) described above wherein said compound activating the G-protein coupled receptor protein according to Section (1) described above is angiotensin, bonbesin, cannabinoid, cholecystokinin, glutamin, serotonin, melatonin, neuropeptide Y, opioid, purine, vasopressin, oxytocin, PACAP, secretin, glucagon, calcitonin; adrenomedulin, somatostatin, GHRH, CRF, ACTH, GRP, PTH, VIP (vasoactive intestinal polypeptide), somatostatin, dopamine, motilin, amylin, bradykinin, CGRP (calcitonin gene-related peptide), leucotriene, pancreastatin, prostaglandine, thromboxane, adenosine, adrenaline, $\alpha$ and $\beta$-chemokine (for example, IL-8, GRO$\alpha$, GRO$\beta$, GRO$\gamma$, NAP-2, ENA-78, PF4, IP10, GCP-2, MCP-1, HC14, MCP-3, I-309, NIP1$\alpha$, MIP-1$\beta$, RANTES and the like), endoserine, enterogastrin, histamine, neurotensin, TRH, pancreatic polypeptide or galanin;

(43) a compound or a salt thereof capable of altering the binding affinity between a ligand and a G-protein coupled receptor protein according to Section (1) described above or a salt thereof which can be obtained by a screening method according to Sections (35) to (42) described above;

(44) a pharmaceutical comprising a compound or a salt thereof capable of altering the binding affinity between a ligand and a G-protein coupled receptor protein according to Section (1) described above or a salt thereof which can be obtained by a screening method according to Sections (35) to (42) described above;

(45) a screening kit according to Section (20) described above comprising a cell comprising a G-protein coupled receptor protein according to Section (1) described above;

(46) a screening kit according to Section (20) described above comprising a membrane fraction of a cell comprising a G-protein coupled receptor protein according to Section (1) described above;

(47) a screening kit according to Section (20) described above comprising a G-protein coupled receptor protein expressed on the cell membrane of a transformant according to Section (11) described above by incubating said transformant;

(48) a compound or a salt thereof capable of altering the binding affinity between a ligand and a G-protein coupled receptor protein according to Section (1) described above or a salt thereof which can be obtained by using a screening kit according to Sections (45) to (47) described above;

(49) a pharmaceutical comprising a compound or a salt thereof capable of altering the binding affinity between a ligand and a G-protein coupled receptor protein according to Section (1) described above or a salt thereof which can be obtained by using a screening kit according to Sections (45) to (47) described above;

(50) a method for quantifying a G-protein coupled receptor protein according to Section (1) described above or a partial peptide according to Section (3) described above or a salt thereof comprising bringing an antibody according to Section (13) described above into contact with the G-protein coupled receptor protein according to Section (1) described above or the partial peptide according to Section (3) described above or a salt thereof;

(51) a method for quantifying a G-protein coupled receptor protein according to Section (1) described above or a partial peptide according to Section (3) described above or a salt thereof in a sample solution comprising a competitive reaction of an antibody according to Section (13) described above with the sample solution and a labeled G-protein coupled receptor protein according to Section (1) described above or partial peptide according to Section (3) described above or a salt thereof followed by a determination of the ratio of the labeled G-protein coupled receptor protein according to Section (1) described above or partial peptide according to Section (3) described above or a salt thereof bound to said antibody; and,

(52) a method for quantifying a G-protein coupled receptor protein according to Section (1) described above or a partial peptide according to Section (3) described above or a salt thereof in a sample solution comprising a simultaneous or sequential reaction of a sample solution with an antibody according to Section (13) described above which is insolubilized on a support and a labeled antibody according to Section (13) described above followed by a determination of the activity of the label on the insolubilized support.

Brief Description of the Drawings

Figure 1 shows a hydrophobicity plot of a G-protein coupled receptor protein according to the invention made based on the amino acid sequence represented by SEQ ID NO.1.

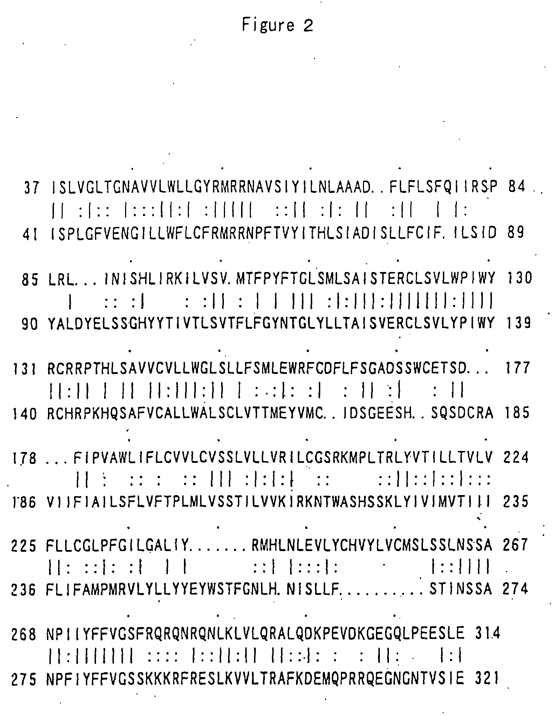

Figure 2 shows the homology of a G-protein coupled receptor protein according to the invention having the amino acid sequence represented by SEQ ID NO.1 when compared with the amino acid sequence of MAS. In this figure, the upper sequence corresponds to the G-protein coupled receptor protein according to the invention and the lower sequence is the amino acid sequence of MAS.

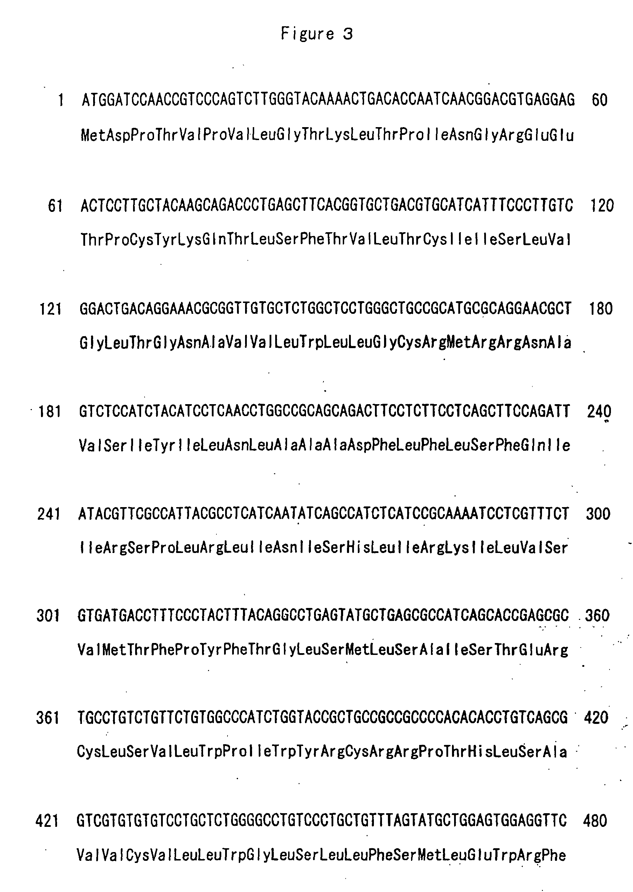

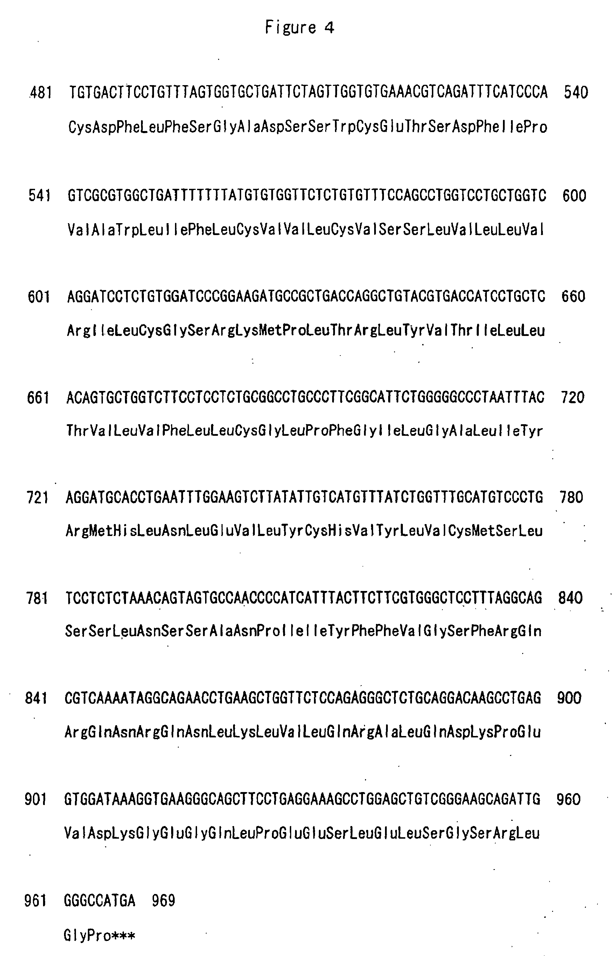

Figure 3 shows the amino acid sequence represented by SEQ ID NO.6 and the base sequence represented by SEQ ID NO.7 (the upper sequence is the base sequence, and the lower sequence is the amino acid sequence) (continued to Figure 4).

Figure 4 shows the amino acid sequence represented by SEQ ID NO.6 and the base sequence represented by SEQ ID NO.7 (the upper sequence is the base sequence, and the lower sequence is the amino acid sequence) (continued from Figure 3).

Best Mode for Carrying out the Invention

A G-protein coupled receptor protein according to the invention (hereinafter sometimes referred to as a receptor protein) is a receptor protein comprising an amino acid sequence identical or substantially identical to the amino acid sequence represented by SEQ ID NO.1.

A receptor protein of the invention may be a protein derived for example from any of various cells (for example, splenic cell, neurocyte, gliacyte, pancreatic β cell, myelocyte, mesangial cell, Langerhans cell, epidermic cell, epithelial cell, endothelial cell, fibroblast, fibrocyte, myocyte, fat cell, immunocyte (e.g., macrophage, T cell, B cell, natural killer cell, mast cell, neutrophile, basophile, eosinophile, monocyte), megakaryocyte, synovial cell, chondrocyte, osteocyte, osteoblast, osteoclast, mammary gland cell, hepatocyte or interstitial cell, or a precursor cell therefor, stem cell or cancer cell and the like) or blood cells of a human or a mammalian animal (for example guinea-pig, rat, mouse, rabbit, swine, sheep, cattle, monkey and the like) or any tissue containing the cells listed above such as brain, brain parts (e. g., olfactory bulb, tonsillar nucleus, cerebral basal bulb, hippocampus, thalamus, hypothalamus, subthalamic nucleus, cerebral cortex, medulla oblongata, cerebellum, occipital lobe, frontal lobe, temporal lobe, putamen, caudatum, blush, nigra), spinal cord, pituitary gland, stomach, pancreas, kidney, liver, gonad, thyroid gland, gallbladder, bone marrow, adrenal gland, skin, muscle, lung, digestive tract (e.g., large intestine, small intestine), blood vessel, heart, thymus, spleen, submandibular gland, peripheral blood, peripheral blood cells, prostate, testis, orchis, ovary, placenta, uterus, bone, joint, skeletal muscle and the like (especially brain and brain parts) as well as a synthetic protein.

An amino acid sequence substantially identical to the amino acid sequence represented by SEQ ID NO.1 may for example be an amino acid having about 50 % or more, preferably about 70 % or more, more preferably about 80 % or more, further preferably about 90% or more, most preferably about 95 % or more of the homology with the amino acid sequence represented by SEQ ID NO.1.

Preferably, a protein comprising an amino acid sequence according to the invention which is substantially identical to the amino acid sequence represented by SEQ ID NO. 1 may for example be a protein having an amino acid sequence substantially identical to the amino acid sequence represented by SEQ ID NO.1 and having an activity substantially similar to the amino acid sequence represented by SEQ ID NO.1.

Typically, a protein comprising an amino acid sequence according to the invention which is substantially identical to the amino acid sequence represented by SEQ ID NO.1 may for example be a protein comprising the amino acid sequence represented by SEQ ID NO.6.

An activity which is substantially similar may for example be ligand binding activity and signal data transmitting activity. The expression "substantially similar" means that the activity is qualitatively similar. Accordingly, the ligand binding activity or the signal data transmitting activity may be different in the degree of the activity or in a quantitative factor such as the molecular weight of the protein, while such activity is preferably comparable (e.g., about 0.01 to 100 times, preferably about 0.5 to 20 times, more preferably 0.5 to 2 times).

While the determination of activity such as ligand binding activity and signal data transmitting activity can be performed in accordance with a method known per se, a ligand determination and a screening method described below may for example be employed.

A receptor protein according to the invention may also be a protein having [1] an amino acid sequence formed as a result of the deletion of one or more (preferably 1 to about 30, more preferably 1 to about 10, most preferably 1 to 5) amino acids in the amino acid sequence represented by Sequence ID. NO. 1, [2] an amino acid sequence formed as a result of the addition of one or more (preferably 1 to about 30, more preferably 1 to about 10, most preferably 1 to 5) amino acids to the amino acid sequence represented by Sequence ID. NO.1, [3] an amino acid sequence formed as a result of the substitution of one or more (preferably 1 to about 30, more preferably 1 to about 10, most preferably 1 to 5) amino acids in the amino acid sequence represented by Sequence ID. NO.1 by other amino acids or [4] an amino acid or a combination thereof.

A receptor protein according to the invention has an N-terminal (amino terminal) in its left end and a C-terminal (carboxyl terminal) in the right end as is ordinary in peptide designation. While a receptor protein of the invention including a receptor protein comprising the amino acid sequence represented by SEQ ID NO.1 as its representative usually has a carboxyl group (-COOH) or a carboxylate (-COO$^-$) at its C-terminal, it may have an amide (-CONH$_2$) or an ester (-COOR) at its C-terminal.

R in an ester employed here may for example be a $C_{1-6}$ alkyl group such as methyl, ethyl, n-propyl, isopropyl and n-butyl, a $C_{3-8}$ cycloalkyl group such as cyclopentyl and cyclohexyl, a $C_{6-12}$ aryl group such as phenyl and α-naphthyl and a $C_{7-14}$ aralkyl group including a phenyl-$C_{1-2}$ alkyl group such as benzyl and phenethyl and an α-naphthyl-$C_{1-2}$ alkyl group such as α-naphthylmethyl as well as a pivaloyloxymethyl group employed widely as an oral ester.

When a receptor protein of the invention has a carboxyl group (or carboxylate) anywhere other than its C-terminal, it may also be included in a receptor protein of the invention when such carboxyl group is amidated or esterified. The ester in such case may be an ester in the C-terminal listed above.

A receptor protein of the invention also includes one whose amino group in the methionine residue at the N-terminal of a protein described above is protected by a protective group (for example, $C_{1-6}$ acyl group including a $C_{2-6}$ alkanoyl group such as formyl and acetyl groups), one whose glutamyl group generated as a result of an in vivo cleavage of the N-terminal is converted into a pyroglutamic acid, one whose substituent on a side chain of an intramolecular amino acid (for example, -OH, -SH, amino group, imidazole group, indole group, guanidino group) is protected by a suitable protective group (for example, $C_{1-6}$ acyl group including a $C_{2-6}$ alkanoyl group such as formyl and acetyl groups), or a conjugated protein such as a glycoprotein having a saccharide chain bound thereto.

Typically, a receptor protein of the invention may for example be a receptor protein derived from a human (preferably derived from a human hippocampus) comprising the amino acid sequence represented by SEQ ID NO.1.

A partial peptide of a receptor protein of the invention (hereinafter sometimes abbreviated as a partial peptide) may be any partial peptide of a receptor protein of the invention described above, and may for example be a site of a receptor protein of the invention which is exposed to the outside of a cell membrane and which has a receptor binding activity.

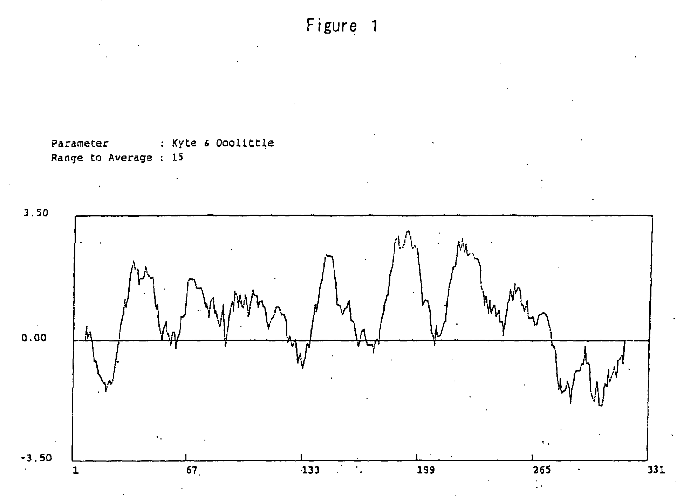

Typically, a partial peptide of the receptor protein having the amino acid sequence represented by SEQ ID NO.1 may for example be a peptide containing a part which is proven to be an extracellular region (hydrophilic site) in a hydrophobicity plot analysis shown in Figure 1. A peptide partially containing a hydrophobic site may also be similarly employed. While a peptide containing each domain discretely may also be employed, a peptide containing several domains simultaneously may also be employed.

The number of the amino acids in a partial peptide of the invention is preferably at least 20, more preferably at least 50 and most preferably at least 100, such amino acids being selected from the constituent amino acids of a receptor protein of the invention.

A substantially identical amino acid sequence means an amino acid sequence having about 50 % or more, preferably about 70 % or more, more preferably about 80 % or more, further preferably about 90 % or more and most preferably about 95 % or more of the homology with the relevant amino acid sequence.

The term "substantially similar activity" employed here has the meaning similar to that described above. A determination of such "substantially similar activity" can be performed as described above.

A partial peptide of the invention may be subjected to the deletion of one or more (preferably 1 to about 10, more preferably 1 to 5) amino acids in an amino acid sequence described above, the addition of one or more (preferably 1 to about 20, more preferably 1 to 10, further preferably 1 to 5) amino acids to the amino acid sequence and the substitution of one or more (preferably 1 to about 10, more preferably several ones, further preferably 1 to 5) amino acids in the amino acid sequence with other amino acids.

While a partial peptide of the invention usually has a carboxyl group (-COOH) or a carboxylate (-COO⁻) at its C-terminal, it may have an amide (-CONH₂) or an ester (-COOR) at its C-terminal similarly to a protein of the invention.

A partial peptide of the invention also includes, similarly to a receptor protein of the invention, one whose amino group in the methionine reside at the N-terminal is protected by a protective group, one whose Gln as a result of an in vivo cleavage at the N-terminal formed is converted into a pyroglutamic acid, one whose substituent on a side chain of an intramolecular amino acid is protected by a suitable protective group, or a conjugated protein such as a glycoprotein having a saccharide chain bound thereto.

While a partial peptide of the invention usually has a carboxyl group (-COOH) or a carboxylate (-COO⁻) at its C-terminal, it may have an amide (-CONH₂) or an ester (-COOR) at its C-terminal similarly to a protein of the invention.

A salt of a receptor protein of the invention or a partial peptide thereof may for example be a physiologically acceptable salt with an acid or a base, and a physiologically acceptable acid addition salt is preferred especially. Such salt may for example be a salt with an inorganic acid (for example, hydrochloric acid, phosphoric acid, hydrobromic acid, sulfuric acid), or with an organic acid (for example, acetic acid, formic acid, propionic acid, fumaric acid, maleic acid, succinic acid, tartaric acid, citric acid, malic acid, oxalic acid, benzoic acid, methanesulfonic acid, benzenesulfonic acid).

A receptor protein of the invention or a salt there of may be produced by a known method for purifying a protein from a cell or a tissue of human or a mammalian animal described above, or may be produced by incubating a transformant comprising a DNA encoding a receptor protein of the invention as described below. Alternatively, a protein synthesis described below or an analogous method may also be employed.

When a tissue or a cell of human or a mammalian animal is used as a starting material, it is homogenized and extracted, for example, with an acid to obtain an extract, which is then purified by a combination of chromatographic methods such as a reverse phase chromatography, an ion exchange chromatography and the like.

A receptor protein or a partial peptide of the invention or its salt or amide may usually be synthesized using a commercial protein synthesis resin. Such resin may for example be a chloromethyl resin, a hydroxymethyl resin, a benzhydrylamine resin, an aminomethyl resin, a 4-benzyloxybenzyl alcohol resin, a 4-methylbenzhydrylamine resin, a PAM resin, a 4-hydroxymethylmethylphenylacetamide resin, a polyacrylamide resin, a 4-(2',4'-dimethoxyphenyl-hydroxymethyl)phenoxy resin, a 4-(2',4'-dimethoxyphenyl-Fmoc aminoethyl)phenoxy resin and the like. Using such resin, an amino acid whose α-amino group and side chain functionalities are suitably protected is condensed on the resin according to any condensation method known per se in the order of the sequence of an intended protein. At the end of the reaction, a protein is isolated from the resin while being deprotected simultaneously, and then subjected to an intramolecular disulfide bond forming reaction in a highly diluted solution to obtain an intended protein or its amide.

While the condensation of a protected amino acid described above may be effected using various activating reagents which can be employed for synthesizing a protein, a carbodiimide is employed preferably. Such carbodiimide may be DCC, N,N'-diisopropylcarbodiimide, N-ethyl-N'-(3-dimethylaminopropyl) carbodiimide and the like. For an activation using any of those listed above, a protected amino acid may be added directly to a resin together with a racemization-inhibiting auxiliary agent (for example, HOBt, HOOBt), or a protected amino acid may previously be activated as a symmetric acid anhydride or an HOBt ester or an HOOBt ester and then added to a resin.

A solvent used in an activation of a protected amino acid or in a condensation with a resin may be one selected from the solvents known to be useful in a protein condensation reaction. Those employed may for example be an acid amide such as N,N-dimethylformamide, N,N-dimethylacetamide arid N-methylpyrrolidone, a halogenated hydrcarbon such as methylene chloride and chloroform, an alcohol such as trifluoroethanol, a sulfoxide such as dimethylsulfoxide, an ether such as pyridine, dioxane and tetrahydrofuran, a nitrile such as acetonitrile and propionitrile, an ester such as methyl acetate and ethyl acetate, or a mixture thereof. The reaction temperature may appropriately be selected from the range known to be useful in a protein binding reaction, and may usually range from -20°C to 50°C. An activated amino acid derivative is employed usually in excess of 1.5 to 4 times. When a ninhydrin reaction test revealed an insufficient condensation, the condensation is repeated without any deprotection to achieve a sufficient condensation. When a repetitive condensation is still not successful in achieving a sufficient condensation, acetic anhydride or acetylimidazole may be employed for acetylating an unreacted amino acid.

A protective group for an amino group of a starting material may for example be Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulfenyl, diphenylphosphonothioyl, Fmoc and the like.

A carboxyl group can be protected for example by an alkylesterification (for example a straight, branched or cyclic alkylesterification employing methyl, ethyl, propyl, butyl, t-butyl, cyclopentyl, cyclohexyl, cycloheptyl, cyclooctyl, 2-adamantyl and the like), an aralkylesterification (for example benzylesterification, 4-nitrobenzylesterification, 4-methoxybenzylesterification, 4-chlorobenzylesterification, benzhydrylesterification), phenacylesterification and can also be protected as benzyloxycarbonylhydrazide, t-butoxycarbonylhydrazide, tritylhydrazide and the like.

The hydroxyl group of serine can be protected for example by an esterification or an etherification. A group suitable in such esterification may for example be a lower alkanoyl group such as an acetyl group, an aroyl group such as a benzoyl group, or a group derivatized from carbonic acid such as a benzyloxycarbonyl group and an ethoxycarbonyl group. A group suitable in such etherification may for example be a benzyl, tetrahydropyranyl and t-butyl groups.

A protective group for a phenolic hydroxyl group of tyrosine may for example be Bzl, $Cl_2$-Bzl, 2-nitrobenzyl, Br-Z, t-butyl and the like.

A protective group for imidazole of histidine may for example be Tos, 4-methoxy-2,3,6-trimethylbenzenesulfonyl, DNP, benzyloxymethyl, Bum, Boc, Trt, Fmoc and the like.

A starting substance whose carboxyl group is activated may for example be a corresponding acid anhydride, an azide, an activated ester [ester with alcohol (for example pentachlorophenol, 2,4,5-trichlorophenol, 2,4-dinitrophenol, cyanomethyl alcohol, p-nitrophenol, HONB, N-hydroxysuccinimide, N-hydroxyphthalimide, HOBt)]. A starting substance whose amino group is activated may for example be a corresponding phosphoryl amide.

A method for a deprotection (cleavage) may be a catalytic hydrogenation under a hydrogen flow in the presence of a catalyst such as Pd-black or Pd-C, a treatment with an acid such as anhydrous hydrofluoric acid, methanesulfonic acid, trifluoromethanesulfonic acid, trifluoroacetic acid or a mixture thereof, a treatment with a base such as diisopropylethylamine, triethylamine, piperidine, piperazine and the like, as well as a reduction with sodium in a liquid ammonia. A cleavage employing an acid treatment described above is performed usually at a temperature of -20°C to 40°C, and such acid treatment is effected advantageously by adding a cation scavenger such as anisol, phenol, thioanisol, m-cresol, p-cresol, dimethylsulfide, 1,4-butanedithiol, 1,2-ethanedithiol and the like. A 2,4-dinitrophenyl group employed as an imidazole protective group for histidine is deprotected by a thiophenol treatment, while a formyl group employed as an indole protective group of tryptophan is removed by a deprotection using an acid treatment in the presence of 1,2-ethanedithiol and 1,4-butandithiol as described above and also by a treatment with an alkali such as a dilute solution of sodium hydroxide and a dilute ammonia.

A protection of a functional group which should no be involved in a reaction of starting materials, a protective group therefor, a deprotection of such protective group and an activation of a functional group involved in a reaction may appropriately be selected from the groups and the methods known per se.

In an alternative method for obtaining an amide of a protein, the α-carboxyl group of the amino acid at the carboxy terminal may for example be protected as being amidated, and then a peptide chain (protein) is elongated to a desired length in the direction of its amino group, and then a protein from which only the protection group of the α-amino group at the N-terminal of the peptide is removed and a protein from which only the protection group of the carboxyl group at the C-terminal thereof is removed are produced, and the both proteins are condensed in a solvent mixture described above. Such condensation is detailed above. After purifying a protected protein obtained by the condensation, all protective groups are removed by the methods described above to yield a desired crude protein. This crude protein can be purified by any of the known purification means and a target fraction is lyophilized to obtain an amide of the desired protein.

For obtaining an ester of a protein, the α-carboxyl group of the amino acid at the carboxy terminal may for example be condensed with a desired alcohol to form an amino acid ester, which is converted into an ester of a desired protein similarly to an amide of a protein.

A partial peptide of a protein of the invention or its salt can be produced according to a peptide synthesis method known per se or by cleaving a protein of the invention with an appropriate peptidase. Such peptide synthesis method may be a solid phase synthesis or a liquid phase synthesis. Thus, a partial peptide or an amino acid capable of constituting a protein of the invention is condensed with the remainder moiety and then a protective group, if any, of the product is removed to obtain an intended peptide. Examples of the known condensation methods and deprotection methods are described in References [1] to [5] shown below.

[1] M.Bodanszky and M.A. Ondetti, Feptide Synthesis, Interscience Publishers, New York (1966);
[2] Schroeder and Luebke, The Peptide, Academic Press, New York (1965);
[3] N.Izumiya et al, Basics and experiments of peptide synthesis, Maruzen (1975);
[4] H.Yajima et al, Biochemisty experiment 1, Protein chemistry IV, 205 (1977);
[5] H.Yajima (supervision), Pharmaceutical development II, Vo1.14, Peptide synthesis, Hirokawa Shoten.

After the reaction, a partial peptide of the invention can be isolated and purified by a combination of a solvent extraction, a distillation, a column chromatography, a liquid chromatography, a recrystallization and the like. When the partial peptide thus obtained is in a free form then it can be converted into a suitable salt by a known method, and when the productis a salt then it can be converted into a free form or other salts by a known method.

A polynucleotide encoding a receptor protein of the invention may be any polynucleotide comprising a base sequence (DNA or RNA, preferably DNA) which encodes the receptor protein of the invention described above. Such polynucleotide may be a DNA or an RNA such as an mRNA which encodes a receptor protein of the invention, and which may be double-stranded or single-stranded. A double-stranded polynucleotide may be a double-stranded DNA, a double-stranded RNA or a hybrid of DNA:RNA. A single-stranded polynucleotide may be a sense (i.e., encoding) strand or an antisense (i.e., non-encoding) strand.

A DNA encoding a receptor protein of the invention may be a genome DNA, a genome DNA library, a cDNA derived from a cell or a tissue described above, a CDNA library derived from a cell or a tissue described above and a synthetic DNA. A vector employed to obtain a library may be a bacteriophage, a plasmid, a cosmid, a phagimid and the like. A total RNA or an mRNA fraction prepared from a cell or a tissue described above may also be used directly in an amplification by a reverse transcriptase polymerase chain reaction (hereinafter abbreviated as RT-PCR).

Typically, a DNA encoding a receptor protein of the invention may for example be a DNA comprising the base sequence represented by SEQ ID NOs.2, 3 or 7 or a DNA having a base sequence capable of being hybridized under a high stringent condition with the base sequence represented by SEQ ID NO.2, 3 or 7 and encoding a receptor protein having an activity substantially similar to that of the protein of the invention (e,g., ligand binding activity, signal data transmitting activity).

A DNA capable of being hybridized with the base sequence represented by SEQ ID NO.2, 3 or 7 may for example be a DNA having about 70 % or more, preferably about 80 % or more, more preferably about 90 % or more and most preferably about 95 % or more of the homology with the base sequence represented by SEQ ID NO.2, 3 or 7.

As evident also from Examples 1 to 3 described below, the presence of a DNA comprising a base sequence represented by SEQ ID NO.2, 3 or 7 suggests a genetic polymorphism, which enables an application to a diagnostic agent described below

A hybridization can be performed by a method known per se or its modification, for example, a method described in Molecular Cloning, 2nd, J. Sambrook et al., Cold Spring Harbor Lab. Press (1989). When a commercial library is employed, an attached instruction may be followed. More preferably, a high stringent condition is employed.

Such high stringent condition may for example be one employing a sodium concentration of about 19 to 40 mM, preferably about 19 to 20 mM and a temperature of about 50 to 70°C, preferably about 60 to 65°C. A sodium concentration of about 19 mM combined with a temperature of about 65°C is most preferred.

More typically, a DNA encoding a receptor protein having the amino acid sequence represented by SEQ ID NO.1 may be a DNA having the base sequence represented by SEQ ID NO.2 or 3, and a DNA encoding a receptor protein having the amino acid sequence represented by SEQ ID NO.6 may be a DNA having the base sequence represented by SEQ ID NO.7.

A polynucleotide comprising a part of the base sequence of a DNA encoding a receptor protein of the invention or a part of the base sequence complementary with such DNA is intended not only to comprise the DNA encoding a partial peptide of the invention described above but also to comprise an RNA.

According to the invention, an antisense polynucleotide (nucleic acid) capable of inhibiting the replication or the expression of a G-protein coupled receptor protein gene can be designed and synthesized based on the base sequence data of a DNA which is cloned or encodes a determined G-protein coupled receptor protein. Such polynucleotide (nucleic acid) can be hybridized with an RNA of a G-protein coupled receptor protein gene whereby inhibiting the synthesis or the function of such RNA, or can interact with a G-protein coupled receptor protein-related RNA whereby regulating or controlling the expression of a G-protein coupled receptor protein gene. By using a polynucleotide complementary with a selected sequence of a G-protein coupled receptor protein-related RNA and a polynucleotide capable of being hybridized specifically with such G-protein coupled receptor protein-related RNA, an in vivo or in vitro determination or regulation of such G-protein coupled receptor protein gene (for controlling an excessive expression or enhancing the expression as desired) can be achieved in accordance with a method known per se. Thus, an application can be made to the diagnosis and the prophylaxis or the therapy for a disease related to the deficiency or the dysfunction of such G-protein coupled receptor protein. The expression "corresponding to" is used herein to mean a homology to or a complementarity with a certain sequence of a nucleotide including a gene, a base or a nucleic acid. The expression "corresponding to" when used here in the context of the relationship of a nucleotide, a base sequence and a peptide (protein) usually means an amino acid of a peptide (protein) to be derived from the nucleotide (nucleic acid) sequence or a sequence complementary therewith. While a 5'-end hairpin loop, a 5'-end 6 base pair repeat, a 5'-end non-coding region, a polypeptide translation initiation codon, a protein-encoding region, an ORF translation initiation codon, a 3'-end non-coding region, a 3'-end palindrome region and a 3'-end hairpin loop of a G-protein coupled receptor protein gene can be selected as a preferred target region, any region in the G-protein coupled receptor protein gene can also be selected as a target region.

The relationship between an intended nucleic acid and a polynucleotide complementary with at least a part of a target region, i.e., the relationship with a polynucleotide capable of being hybridized with a target can be regarded to be "antisense". An antisense polynucleotide may for example be a polydeoxynucleotide containing 2-deoxy-D-ribose, a polydeoxynucleotide containing. D-ribose, a polynucleotide of any other type which is an N-glycoside of a purine or pyrimidine base, any other polymer containing a non-nucleotide backbone (for example, a commercially available protein nucleic acid and a synthetic sequence-specific nucleic acid polymer) or any other polymer containing a special binding (provided that such polymer contains a nucleotide having a configuration capable of accepting a base pairing or a base adhesion found in a DNA or an RNA). It may be a double-stranded DNA, a single-stranded DNA, a double-stranded RNA, a single-stranded RNA, a DNA:RNA hybrid, a non-modified polynucleotide (or a non-modified oligonucleotide), a polynucleotide having a known modification, for example, one having a known label, a capped polynucleotide, a methylated polynucleotide, a polynucleotide obtained by substituting one or more natural nucleotides with analogues, a polynucleotide whose intramolecular nucleotides are modified, for example, one having a non-charged bond (for example, methylphosphonate, phosphotriester, phosphoramidate, carbamate), one having a charged bond or a sulfur-containing bond (for example, phosphorothioate, phosphorodithioate), for example, one having a side chain residue such as a protein (nuclease, nuclease inhibitor, toxin, antibody, signal peptide, poly-L-lysine) or a saccharide (for example, monosaccharide), one having an intercalating compound (for example, acridine, psoralen), one containing a chelating compound (for example, metal, radioactive metal, boron, oxidativemetal), one containing an alkylating agent, one having a modified bond (for example, $\alpha$-anomer nucleic acid) and the like. The terms "nucleoside", "nucleotide" and "nucleic acid" employed here may include a form containing not only purine and pyrimidine bases but also other modified heterocyclic bases. Such modified form may contain methylated purine and pyrimidine, acylated purine and pyrimidine as well as other heterocyclic rings. A modified nucleoside and a modified nucleotide may be modified further at their saccharide moieties, for example, as a result of the substitution of one or more hydroxyl groups by halogens or aliphatic groups, or as a result of the conversion into a functional group such as an ether or an amine.

An antisense polynucleotide (nucleic acid) of the invention is an RNA, a DNA, or a modified nucleic acid (RNA, DNA). Typically, such modified nucleic acid may for example be a sulfur derivative or a thiophosphate derivative of a nucleic acid and those which are resistant to a polynucleosideamide or oligonucleosideamide degradation, others may also be employed. An antisense nucleic acid of the invention can preferably be designed based on the policy described below. Thus, an effort is made for the purpose of achieving a higher intracellular stability of an antisense nucleic acid, a higher cellular permeability of an antisense nucleic acid, a higher affinity with a target sense strand, and a lower toxicity, if any, of an antisense nucleic acid.

Such modifications are reported extensively in this field, as disclosed for example in J. Kawakami et al., Pharm Tech Japan, Vol.8, pp.247, 1992; Vol.8, pp.395, 1992; S.T. Crooke et al. ed., Antisense Research and Applications, CRC Press, 1993.

An antisense nucleic acid of the invention can be imparted with a variation or may contain a modified saccharide, base or bond, and can be provided in a special form such as a liposome or a microsphere, applied to an gene therapy or can be given as an adduct. Such adduct may be a polycation such as a polylysine which serves to neutralize the electric charge of a phosphate backbone or a hydrophobic material such as a lipid (for example, phospholipid, cholesterol) which serves to enhance the interaction with a cell membrane and to promote the intake of a nucleic acid. A lipid to be added preferably is cholesterol or a derivative thereof (for example, cholesteryl chloroformate, cholic acid) and the like. These are capable of being attached to the 3'- or 5'-end of a nucleic acid, and can be attached via a base, a saccharide or an intracellular nucleoside bond. Other groups may for example be a group for a capping located specifically at the 3'- or 5'-end of a nucleic acid, such as those intended to avoid the degradation by a nuclease such as exonucleases or RNases. A group for such capping may be a protective group for a hydroxyl group known in the art such as a glycol including polyethylene glycol and tetraethylene glycol, which are not limiting.

The inhibitory activity of an antisense nucleic acid can be examined using a transformant of the invention, an in vivo or in vitro gene expression system of the invention or an in vivo or in vitro translation system of a G-protein coupled receptor protein. Such nucleic acid can be applied to a cell by a method known per se.

A DNA encoding a partial peptide of the invention may be any DNA containing a base sequence encoding a partial peptide of the invention described above. It may be a genome DNA, a genome DNA library, a cDNA derived from a cell or a tissue described above, a cDNA library derived from a cell or a tissue described above and a synthetic DNA. A vector employed to obtain a library may be a bacteriophage, a plasmid, a cosmid, a phagimid and the like. An mRNA fraction prepared from a cell or a tissue described above may also be used directly in an amplification by a reverse transcriptase polymerase chain reaction (hereinafter abbreviated as RT-PCR).

Typically, a DNA encoding a partial peptide of the invention may for example be [1] a DNA having a partial base sequence of a DNA comprising the base sequence represented by SEQ ID NOs.2, 3 or 7 or [2] a DNA having a partial base sequence of a DNA having a base sequence capable of being hybridized under a high stringent condition with the base sequence represented by SEQ ID NO.2, 3 or 7 and encoding a receptor protein having an activity substantially similar to that of the protein of the invention (e.g., ligand binding activity, signal data transmitting activity).

A DNA capable of being hybridized with the base sequence represented by SEQ ID NO.2, 3 or 7 may for example be a DNA having about 70 % or more, preferably about 80 % or more, more preferably about 90 % or more and most preferably about 95 % or more of the homology with the base sequence represented by SEQ ID NO.2, 3 or 7.

In a method for cloning a DNA which completely encodes a receptor protein or a partial peptide thereof according to the invention (hereinafter abbreviated as a receptor protein of the invention) a synthetic DNA primer having a partial base sequence of the receptor protein of the invention is employed in an amplification by a PCR, or a selection is made by means of a hybridization of a DNA integrated into a suitable vector and labeled with a DNA fragment encoding a part or all of the receptor protein of the invention or with a synthetic DNA. A hybridization can be performed for example by a method described in Molecular Cloning, 2nd, J.Sambrook et al., Cold Spring Harbor Lab. Press (1989). When a commercial library is employed, an attached instruction may be followed.

The conversion of the base sequence of a DNA can be performed by a method known per se such as Gapped duplex method or Kunkel method or its modification using a known kit, such as Mutan™-G (Takara) or Mutan™-K (Takara).

A cloned receptor protein-encoding DNA can be used directly or after a digestion with a restriction enzyme or an addition of a linker if desired. Such DNA may have an ATG as a translation initiation codon at its 5' terminal and a TAA, TGA or TAG as a translation termination codon at its 3' terminal. Such translation initiation codon or termination codon may be added using an appropriate synthetic DNA adapter.

An expression vector for a receptor protein of the invention can be prepared for example by (i) cutting a desired DNA fragment out of a DNA encoding the receptor protein of the invention, and (ii) ligating said DNA fragment to the downstream of a promoter of a suitable expression vector.

Such vector may for example be an E.coli-derived plasmid (e.g., pBR322, pBR325, pUC12, pUC13), a B. subtilis-derived plasmid (e.g., pUB110, pTP5, pCI94), a yeast-derivedplasmid (e.g., pSH19, pSH15), abacteriophage such as λ phage, an animal virus such as retrovirus, vaccinia virus, baculovirus and the like, as well as pA1-11, pXT1, pRc/CMV, pRc/RSV, pcDNAI/Neo and the like.

A promoter employed in the invention may be any promoter which is appropriate correspondingly to a host employed for expressing a gene. For example, when an animal cell is employed as a host cell, those exemplified are SRα promoter, SV40 promoter, LTR promoter, CMV promoter, HSV-TK promoter and the like.

Among those listed above, CMV promoter and SRα promoter are employed preferably. Those preferred for an Escherichia microorganism as a host cell are trp promoter, lac promoter, recA promoter, λP$_L$ promoter, lpp promoter and the like, those preferred for a Bacillus microorganism as a host cell are SPO1 promoter, SPO2 promoter, penP promoter and the like, those preferred for a yeast as a host cell are PHO5 promoter, PGK promoter, GAP promoter, ADH promoter and the like. Those preferred for an insect cell as a host cell are polyhedrin promoter, p10 promoter and the like.

In addition to those described above, an expression vector containing, if desired, an enhancer, a splicing signal, a polyA addition signal, a selection marker, an SV 40 replication origin (hereinafter sometimes abbreviated as SV40ori) and the like may also be employed. A selection marker may for example be a dihydrofolic acid reductase (hereinafter sometimes abbreviated as dhfr) gene [methotrexate (MTX) resistant], an ampicillin resistant gene (hereinafter sometimes abbreviated as Amp$^r$), a neomycin resistant gene (hereinafter sometimes abbreviated as Neo$^r$, G418 resistant) and the like. Especially when a dhfr gene is employed as a selection marker using a CHO (dhfr⁻) cell, an intended gene can be selected using a thymidine-free medium.

A signal sequence suitable for a host cell may also be added if necessary to the N-terminal of a receptor protein of the invention. Those preferred for an Escherichia microorganism as a host cell are Pho A·signal sequence, Omp A·signal sequence and the like, those preferred for a Bacillus microorganism as a host cell are α-amylase·signal sequence, subtilicin·signal sequence and the like, those preferred for a yeast as a host cell are MFα·signal sequence, SUS2·signal sequence and the like, and those preferred for an animal cell as a host cell are insulin·signal sequence, α-interferon·signal sequence, an antibody molecule·signal sequence and the like.

Using a vector comprising a DNA encoding a receptor protein of the invention thus constructed, a transformant can be prepared.

A host cell may for example be an Escherichia microorganism, a Bacillus microorganism, a yeast, an insect cell, an insect, an animal cell and the like.

Such Escherichia microorganism may for example be Escherichia coli K12·DH1 (Proc. Natl. Acad. Sci. USA, Vol. 60, 160 (1968)), JM103 (Nucleic Acids Research, Vol.9, 309 (1981)), JA221 (Journal of Molecular Biology, Vol. 120, 517 (1978)), HB101 (Journal of Molecular Biology, Vol.41, 459 (1969)), C600 (Genetics, Vol.39, 440 (1954)) and the like.

A Bacillus microorganism may for example be Bacillus subtilis MI114 (Gene, Vol.24, 255 (1983)), 207-21 (Journal of Biochemistry, Vol.95, 87 (1984)) and the like.

A yeast may for example be Saccharomyces cerevisiae AH22, AH22R⁻, NA87-11A, DKD-5D, 20B-12, Schizosaccharomyces pombe NCYC1913, NCYC2036, Pichia pastoris KM71 and the like.

An insect cell, when a virus is AcNPV, may for example be an armyworm-derived cultured cell line, (Spodoptera frugiperda cell; Sf cell), a Trichoplusia ni mesenteron-derived MG1 cell, Trchoplusia ni egg-derived High Five™ cell, Mamestrabrassicae-derived cell, Estigmena acrea-derived cell and the like. A cell when a virus is BmNPV may for example be a silkworm-derived cultured cell line (Bombyx mori N cell; BmN cell) and the like. Such Sf cell may for example be an Sf9 cell (ATCC CRL1711), an Sf21 cell (for both, see Vaughn, J.L. et al., In Vivo, 13, 213-217 (1977)) and the like.

An insect may for example be a larva of a silkworm (Maeda et al., Nature, Vol.315, 592 (1985)).

An animal cell may for example be a simian cell COS-7, V ero, a Chinese hamster cell CHO (hereinafter abbreviated as CHO cell), a dhfr gene-defect Chinese hamster cell CHO (hereinafter abbreviated as CHO (dhfr⁻) cell), amouseLcell, a mouse AtT-20, a mouse myeloma cell, a rat GH3, a human FL cell and the like.

An Escherichia microorganism can be transformed for example by a method described in Proc. Natl. Acad. Sci. USA, Vol. 69, 2110 (1972), or Gene, Vol.17, 107 (1982). A Bacillus microorganism can be transformed for example by a method described in Molecular and General Genetics, Vol.168, 111 (1979).

A yeast can be transformed for example by a method described in Methods in Enzymology, Vol. 194, 182-187 (1991), Proc. Natl. Acad. Sci. USA, Vol.75, 1929 (1978) and the like.

An insect cell or an insect can be transformed for example by a method described in Bio/Technology, 6, 47-55 (1988).

An animal cell can be transformed for example by a method described in CELL ENGINEERING EXTRA ISSUE No.8, NEW CELL ENGINEERING PROTOCOL, 263-267 (1995) (SHUJUNSHA), Virology, VI.52, 456 (1973) and the like.

As described above, a transformant which had been transformed with an expression vector comprising a DNA encoding a G-protein coupled receptor protein can be obtained.

When incubating a transformant whose host cell is an Escherichia microorganism or a Bacillus microorganism, a suitable culture medium employed is a liquid medium which may contain substances required for the growth of the relevant transformant such as carbon sources, nitrogen sources, inorganic substances and the like. A carbon source may for example be glucose, dextrin, soluble starch, sucrose and the like, and a nitrogen source may for example be an inorganic or organic material such as an ammonium salt, a nitrate, corn steep liquor, peptone, casein, meat extract, soybean bran, potato extract and the like, and an inorganic substance may for example be calcium chloride, sodium dihydrogen phosphate, magnesium chloride and the like. A yeast, a vitamin and a growth promoting factor may also be added. The pH of a medium is preferably about 5 to 8.

A preferred culture medium for incubating an Escherichia microorganism may for example be a M9 medium containing glucose and casamino acid (Miller, Journal of Experiments in Molecular Genetics, 431-433, Cold Spring Harbor Laboratory, New York (1972)). If necessary, a medium may contain an agent for facilitating the action of a promoter such as 3β-indolylacrylic acid. When an Escherichia microorganism is employed as a host cell, the incubation is performed usually at 15 to 43°C for about 3 to 24 hours, if necessary with aeration or stirring.

When a Bacillus microorganism is employed as a host cell, the incubation is performed usually at 30 to 40°C for about 6 to 24 hours, if necessary with aeration or stirring.

When incubating a transformant whose host cell is a yeast, a suitable culture medium employed may for example be a Burkholder minimum medium (Bostian, K.L. et al., Proc. Natl. Acad. Sci. USA, Vol. 77, 4505 (1980), and a 0.5 % casamino acid-supplemented SD medium (Bitter, G.A. etal., Proc. Natl. Acad. Sci. USA, Vol.81, 5330 (1984). The pH of a culture medium is adjustedpreferably at about 5 to 8. The incubation is performed usually at 20 to 35°C for about 24 to 72 hours, if necessary with aeration or stirring.

When incubating a transformant whose host cell is an insect cell or an insect, a suitable culture medium employed may for example be Grace's Insect Medium (Grace, T.C.C., Nature, 195, 788 (1962) supplemented appropriately, for example, with an inactivated 10 % bovine serum. The pH of the culture medium is adjusted preferably at about 6.2 to 6.4. The incubation is performed usually at 27°C for about 3 to 5 days, if necessary with aeration or stirring.

When incubating a transformant whose host cell is an animal cell, a suitable culture medium employed may for example be a MEM medium supplemented with about 5 to 20 % fetal bovine serum (Science, Vol.122, 501 (1952), a DMEM medium (Virology, Vol. 8, 396 (1959), an RPMI1640 medium (The Journal of the American Medical Association, Vol.199, 519 (1967), a 199 medium (Proceedings of the Society for the Biological Medicine, Vol.73, 1 (1950), and the like. The pH of a culture medium is preferably about 6 to 8. The incubation is performed usually at 30 to 40°C for about 15 to 60 hours, if necessary with aeration or stirring.

As described above, a G-protein coupled receptor protein according to the invention can be produced in a cell membrane of a transformant.

In order to separate and purify a receptor protein of the invention from a cell culture described above, a method described below may for example be employed.

For extracting a receptor protein of the invention from a cultured microorganism or a cultured cell, the cell is collected by a known method after incubation and suspended in a suitable buffer solution, which is subjected to. ultrasonication, treatment with lysozyme and/or a freezing and thawing cycle to destroy the cell followed by centrifugation or filtration to obtain a crude extract of. the receptor protein. The buffer solution may contain a protein modifier such as urea or guanidine hydrochloride or a surfactant such as Triton X-100™. When a receptor protein is secreted into a culture medium, then a cell and a supernatant are separated by a method known per se after completing the incubation to collect the supernatant.

The purification of a receptor protein contained in a culture supernatant or an extract thus obtained may be performed by an appropriate combination of separation and purification methods known per se. Such known separation and purification methods are a method utilizing solubility such as salting out or solvent precipitation, a methodmainly utilizing the difference in the molecular weight such as dialysis, ultrafiltration, gel filtration and SDS-polyacrylamide gel electrophoresis, a method utilizing the difference in the electric charge such as ion exchange chromatography, a method utilizing the specific affinity such as affinity chromatography, a method utilizing the difference in the hydrophobicity such as reverse phase high pressure liquid chromatography, a method utilizing the difference in the isoelectric point such as isoelectric focusing and the like.

When a receptor protein thus obtained is in a free form then it can be converted into a salt by a method known per se or its modification, and, on the contrary, when it is obtained as a salt then it can be converted into a free form or another salt by a method known per se or its modification.

It is also possible that a receptor protein produced by a recombinant is treated with a suitable protein-modifying enzyme before or after a purification to achieve a desired modification or a partial removal of a polypeptide. Such protein-modifying enzyme may for example be trypsin, chymotrypsin, arginylendopeptidase, protein kinase, glycosidase and the like.

A receptor protein of the invention or its salt thus produced can be examined for its activity for example by a labeled ligand binding test or an enzyme immunoassay employing a specific antibody.

An antibody against a receptor protein or a partial peptide of the invention or its salt may be either a polyclonal antibody or a monoclonal antibody provided that it can recognize such receptor protein or partial peptide of the invention or its salt.

An antibody against a receptor protein or a partial peptide of the invention or its salt (hereinafter abbreviated as a receptor protein of the invention) can be produced using the receptor protein of the invention as an antigen by a known method for producing an antibody or antiserum.

[Monoclonal antibody preparation]

(a) Preparation of monoclonal antibody-producing cell

A receptor protein of the invention can be administered as it is or in combination with a carrier or diluent to a site where the antibody can be produced in response to the administration to a mammalian animal. The administration may be combined with an administration of a Freund's complete adjuvant or a Freund's incomplete adjuvant for the purpose of enhancing the antibody-producing ability. The administration is performed usually once per 2 to 6 weeks, 2 to 10 times in total. A mammalian animal employed may for example be monkey, rabbit, dog, guinea-pig, mouse, rat, sheep and goat, with mouse and rat being employed preferably.

For preparing a monoclonal antibody-producing cell, antigen-immunized warm-blooded animals, for example mice, are screened for an individual exhibiting an antibody titre, from which a spleen or a lymph node is extracted 2 to 5 days after the final immunization, and an antibody-producing cell contained therein is fused with a myeloma cell, whereby preparing a monoclonal antibody-producing hybridoma. The antibody titre of an antiserum can be determined for example by reacting a labeled receptor protein described below with the antiserum followed by determining the activity of the label bound to an antibody. The fusion can be accomplished by a known method such as one by Kohler and Milstein (Nature, 256, 495 (1975)). A fusion promoting agent may for example be a polyethylene glycol (PEG) or Sendai virus, with PEG being employed preferably.

A myeloma cell may for example be NS-1, P3U1, SP2/0, with P3U1 being employed preferably. A preferred ratio of the antibody-producing cell count (spleen cell count) and the myeloma cell count employed is about 1:1 to 20: 1, and an efficient cell fusion is accomplished by adding a PEG (preferably PEG 1000 to PEG 6000) at a concentration of 10 to 80 % and incubating at 20 to 40°C, preferably 30 to 37° C, for 1 to 10 minutes.

While various methods are applicable in screening for a monoclonal antibody-producing hybridoma, those which may be exemplified are a method involving an addition of a hybridoma culture supernatant to a solid phase (e. g., a microplate) on which a antigen such as a receptor protein is adsorbed directly or in combination with a carrier followed by an addition of an anti-immunoglobulin labeled with a radioactive substance or with an enzyme (anti-mouse immunoglobulin antibody is employed when the cell employed in the cell fusion is a mouse cell) or protein A whereby detecting a monoclonal antibody bound to the solid phase, or a method involving an addition of a hybridoma culture supernatant to a solid phase on which an anti-immunoglobulin antibody or protein A is absorbed followed by an addition of a receptor protein labeled with a radioactive substance or with an enzyme whereby detecting a monoclonal antibody bound to the solid phase.

While a monoclonal antibody can be selected by a method known per se or its modification, a HAT (hypoxanthine, aminopterin, thymidine)-supplemented medium for an animal cell culture is usually employed. A medium for selection and breeding may be any medium capable of growing a hybridoma. For example, an RPMI 1640 medium supplemented with 1 to 20 %, preferably 10 to 20 % fetal bovine serum, a GIT medium (Wako Pure Chemical) supplemented with 1 to 10 % fetal bovine serum and a serum-free medium for incubating a hybridoma (SFM-101, NISSUI SEIYAKU) may be employed. The incubation temperature is usually 20 to 40°C, preferably about 37°C. The incubation time is usually 5 days to 3 weeks, preferably 1 week to 2 weeks. The incubation may be performed usually under an atmosphere of 5% $CO_2$ gas. The antibody titre of a hybridoma incubation supernatant can be determined similarly to an antibody titre of an antiserum described above.

(b) Purification of monoclonal antibody

A monoclonal antibody can be separated and purified, similarly to a standard method for separating and purifying a polyclonal antibody, such as a method for separating and purifying an immunoglobulin [e.g., salting out, alcohol precipitation, isoelectric precipitation, electrophoresis, ion exchanger (e.g., DEAE) adsorption and desorption, ultracentrifugation, gel filtration, a specific purification for collecting an antibody exclusively using an antigen-binding solid phase or an active adsorbent such as protein A or protein G followed by dissociating the binding to obtain the antibody].

[Polyclonal antibody preparation]

A polyclonal antibody of the invention can be prepared by a method known per se or its modification. For example, a complex of an immune antigen (antigen such as a receptor protein) with a carrier protein is produced and used as described above in the section of the monoclonal antibody preparation to immunize a mammalian animal, from which a material containing an antibody against a receptor protein of the invention is isolated and purified to obtain an antibody.

With regard to a complex of an immune antigen with a carrier protein employed for immunizing amammalian animal, the type of the carrier protein and the mixing ratio of the carrier and a hapten may vary provided that the antibody can be produced efficiently in relation to the hapten crosslinked to the carrier for the immunization, and any substance can be crosslinked at any ratio, and, in a typical method, about 0.1 to 20, preferably about 1 to 5 of parts by weight of bovine serum albumin, bovine thyroglobulin or keyhole limpet hemocyanin is coupled to 1 part by weight of a hapten.

For coupling a hapten to a carrier, various condensing agent can be employed, such as glutaraldehyde or carbodiimide, a maleimide activated ester, an active ester reagent having a thiol group or a dithiopyridyl group.

A condensation product may can be administered as it is or in combination with a carrier or diluent to a site of a warm-blooded animal where the antibody can be produced. The administration may be combined with an administration of a Freund's complete adjuvant or a Freund's incomplete adjuvant for the purpose of enhancing the antibody-producing ability. The administration is performed usually once per 2 to 6 weeks, 3 to 10 times in total.

A polyclonal antibody can be collected from blood, ascites, preferably from blood, of a warm-blooded animal immunized as described above.

The polyclonal antibody titre of an antiserum can be determined similarly to the measurement of the antibody titre of an antiserum described above. A polyclonal antibody can be isolated and purified in accordance with a method for isolating and purifying an immunoglobulin similar to a method for isolating and purifying a monoclonal antibody described above.

A receptor protein of the invention or a salt thereof, a partial peptide there of or a salt thereof and a DNA encoding such receptor protein or a partial peptide thereof can be used in (1) the determination of a ligand (agonist) for a G-protein coupled receptor protein of the invention, (2) a preventing and/or treating agent against a disease related to the dysfunction of a G-protein coupled receptor protein of the invention, (3) a gene diagnostic agent, (4) a method for screening a compound capable of altering the expression level of a receptor protein of the invention or a partial peptide thereof, (5) a preventing and/or treating agent against various diseases containing a compound capable of altering the expression level of a receptor protein of the invention or a partial peptide thereof, (6) a method for quantifying a ligand for a G-protein coupled receptor protein of the invention, (7) a method for screening a compound (agonist, antagonist) capable of altering the binding affinity between a G-protein coupled receptor protein of the invention and a ligand, (8) a preventing and/or treating agent against various diseases containing a compound (agonist, antagonist) capable of altering the binding affinity between a G-protein coupled receptor protein of the invention and a ligand, (9) a quantification of a receptor protein of the invention or a partial peptide thereof or a salt thereof, (10) a method for screening a compound capable of altering the amount of a receptor protein of the invention or a partial peptide thereof in a cell membrane, (11) a preventing and/or treating agent against various diseases containing a compound capable of altering the amount of a receptor protein of the invention or a partial peptide thereof in a cell membrane, (12) the neutralization of a receptor protein of the invention or a partial peptide thereof or a salt thereof by an antibody, and (13) the creation of a non-human animal having a DNA encoding a G-protein coupled receptor protein of the invention.

Especially by using a receptor binding assay system employing an inventive recombinant G-protein coupled receptor protein expression system, a compound (e.g., agonist and antagonist) altering the binding affinity of a ligand to a human- or mammal-specific G-protein coupled receptor can be screened and such agonist or antagonist can be employed in a preventing or treating agent against any relevant disease.

The uses of a receptor protein of the invention or a partial peptide or a salt thereof (hereinafter sometimes abbreviated as a receptor protein and the like of the invention), a DNA encoding a receptor protein of the invention or a partial peptide or a salt thereof (hereinafter sometimes abbreviated as a DNA of the invention) and an antibody directed to a receptor protein and the like of the invention (hereinafter sometimes abbreviated as an antibody of the invention) are discussed below.

(1) Determination of ligand (agonist) for G-protein coupled receptor protein of the invention

A receptor protein of the invention or a salt thereof or a partial peptide of the invention or a salt thereof is useful as a reagent for searching for or determining a ligand (agonist) for a receptor protein of the invention or a salt thereof.

Thus, the present invention provides a method for determining a ligand for a receptor protein of the invention comprising bringing a receptor protein of the invention or a salt thereof or a partial peptide of the invention or a salt thereof into contact with a test compound.

Such test compound may for example be a known ligand (for example, angiotensin, bonbesin, cannabinoid, cholecystokinin, glutamin, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene-related peptide), leucotriene, pancreastatin, prostaglandine, thromboxane, adenosine, adrenaline, $\alpha$ and $\beta$-chemokine (for example, IL-8, GRO$\alpha$, GRO$\beta$, GRO$\gamma$, NAP-2, ENA-78, PF4, IP10, GCP-2, MCP-1, HC14, MCP-3, I-309, NIP1$\alpha$, MIP-1$\beta$, RANTES and the like), endoserine, enterogastrin, histamine, neurotensin, TRH, pancreatic polypeptide or galanin) as well as an extract of a tissue of a human or a mammalian animal (for example, mouse, rat, swine, cattle, sheep and monkey) and a cell culture supernatant. For example, such tissue extract of cell culture supernatant may be added to a receptor protein of the invention and fractionated while determining a cell stimulating activity and the like, whereby finally obtaining a single ligand.

Typically in a ligand determination method of the invention, a receptor protein or a partial peptide thereof is employed, or a recombinant receptor protein expression system is constructed and used in a receptor binding assay system, whereby determining a compound (for example, peptide, protein, non-peptide compound, synthetic compound, fermented compound and the like) or a salt thereof having a cell stimulating activity (for example, an activity which promotes or suppresses arachidonic acid release, acetylcholin release, intracellular $Ca^{2+}$ release, intracellular cAMP production, intracellular cGMP production, inositol phosphate production, cell membrane potential variation, intracellular protein phosphorylation, c-fos activation, pH reduction and the like) as being bound to the receptor protein of the invention.

A ligand determination method of the invention is characterized by a determination of the binding of a test substance to a receptor protein of the invention or a partial peptide thereof or the cell stimulating activity when bringing such receptor protein of the invention or the partial peptide thereof into contact with such test compound.

More typically, the present invention provides:

[1] a method for determining a ligand for a receptor protein of the invention or a salt thereof comprising determining the level of the binding of a labeled test compound to the receptor protein of the invention or the salt thereof or a partial peptide of the invention or a salt thereof when bringing such labeled test compound into contact with such protein or the salt thereof or such partial peptide or the salt thereof;

[2]a method for determining a ligand for a receptor protein of the invention or a salt thereof comprising determining the level of the binding of a labeled test compound to a cell containing the receptor protein of the invention or a membrane fraction of such cell when bringing such labeled test compound into contact with such cell or such membrane fraction;

[3] a method for determining a ligand for a receptor protein of the invention comprising determining the level of the binding of a labeled test compound to the receptor protein expressed on a cell membrane by incubating a transformant containing a DNA encoding the receptor protein of the invention when bringing such labeled test compound into contact with such receptor protein or a salt thereof;

[4]a method for determining a ligand for a receptor protein of the invention or a salt thereof comprising determining a receptor protein-mediated cell stimulating activity (for example, an activity which promotes or suppresses arachidonic acid release, acetylcholin release, intracellular $Ca^{2+}$ release, intracellular cAMP production, intracellular cGMP production, inositol phosphate production, cell membrane potential variation, intracellular protein phosphorylation, c-fos activation, pH reduction and the like) when bringing a labeled test compound into contact with a cell containing the receptor protein of the invention; and,

[5] a method for determining a ligand for a receptor protein of the invention or a salt thereof comprising determining a receptor protein-mediated cell stimulating activity (for example, an activity which promotes or suppresses arachidonic acid release, acetylcholin release, intracellular $Ca^{2+}$ release, intracellular cAMP production, intracellular cGMP production, inositol phosphate production, cell membrane potential variation, intracellular protein phosphorylation, c-fos activation, pH reduction and the like) when bringing a labeled test compound into contact with a receptor protein expressed on a cell membrane by incubating a transformant containing a DNA encoding the receptor protein of the invention.

It is preferred particularly to perform the tests [1] to [3] described above to ensure the binding of a test compound to a receptor protein of the invention prior to performing the tests [4] to [5] described above.

While a receptor protein employed primarily in a method for determining a ligand may be any of the receptor proteins of the invention or those containing a partial peptide of the invention described above, a receptor protein expressed in a large amount using an animal cell is suitable.

In order to produce a receptor protein of the invention, an expression method described above may be employed, and it is preferred to express a DNA encoding such receptor protein in a mammalian cell or an insect cell. While a complementary DNA is employed usually as a DNA fragment encoding a target protein moiety, it is not limiting. For example, a gene fragment or a synthetic DNA may also be employed. For the purpose of introducing a DNA fragment encoding a receptor protein of the invention into an animal host cell to effect a highly efficient expression, it is preferred to integrate such DNA fragment into the downstream of a polyhedron promoter of a nuclear polyhedrosis virus (NPV) classified as a baculovirus whose host is an insect, an SV 40-derived promoter, a retrovirus promoter, a metallothioneine promoter, a human heat shock promoter, a cytomegalovirus promoter, an SRα promoter and the like. An expressed receptor can be examined for its quantity or quality by a method known per se. For example, a method known in a literature (Nambi, P. et al., J. Biol. Chem., Vo. 267, p.19555 to 19559, 1992) may be employed.

Accordingly, in a method for determining a ligand of the invention, those containing a receptor protein of the invention or a partial peptide or a salt thereof may be a receptor protein or a partial peptide or a salt thereof which was purified by a method known per se or a cell containing such receptor protein or a cell membrane fraction obtained therefrom.

When a cell containing a receptor protein of the invention is employed in a method for determining a ligand of the invention, this cell may be immobilized by glutaraldehyde or formalin. Such immobilization can be effected by a method known per se.

A cell containing a receptor protein of the invention is a host cell expressing the receptor protein of the invention, and such host cell may be an Escherichia microorganism, a Bacillus microorganism, a yeast, an insect cell, an animal cell and the like.

A cell membrane fraction means a cell membrane-rich fraction obtained by a method known per se after pelletizing the cell. A cell may be pelletized for example by a method in which a cell is pressed and crushed by a Potter-Elvehjem homogenizer, by using a whirling blender or a polytron (Kinematica), by means of an ultrasonic treatment, or by a method in which a cell is sprayed via a fine nozzle while being pressurized by a French press. A cell membrane may be fractionated mainly by a centrifugal fractionation such as a fractional centrifugation or a density gradient centrifugation. For example, a cell pellet is centrifuged at a low speed (500 rpm to 3000 rpm) for a short period (usually about 1 minute to 10 minutes) to obtain a supernatant, which is then centrifuged at a higher speed (15000 rpm to 30000 rpm) usually for 30 minutes to 2 hours to obtain a pellet, which is used as a membrane fraction. This membrane fraction contains a large amount of the membrane components such as an expressed receptor protein and phospholipids and membrane proteins derived from the cell.

The amount of a receptor protein in a cell containing such receptor protein or a membrane fraction thereof is preferably $10^3$ to $10^8$ molecules per cell, more preferably $10^5$ to $10^7$ molecules. A higher expression leads to a higher ligand binding activity (specific activity) per membrane fraction, whereby allowing not only a highly sensitive screening system to be established but also a large amount of a sample to be determined in an identical lot.

For performing the methods (1) to (3) described above for determining a ligand for a receptor protein of the invention or a salt thereof, a suitable receptor protein fraction and a labeled test compound are required.

A receptor protein fraction is preferably a natural receptor protein fraction or a recombinant receptor fraction having an activity which is equivalent to that of the natural one. The expression "activity which is equivalent" employed here means an equivalent ligand binding activity or signal data transmission activity.

A labeled test compound may for example be $[^3H]$-, $[^{125}I]$-, $[^{14}C]$- or $[^{35}S]$-labeled angiotensin, bonbesin, cannabinoid, cholecystokinin, glutamin, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene-related peptide), leucotriene, pancreastatin, prostaglandine, thromboxane, adenosine, adrenaline, α and β-chemokine (for example, IL-8, GROα, GROβ, GROγ, NAP-2, ENA-78, PF4, IP10, GCP-2, MCP-1, HC14, MCP-3, I-309, MIP1α, MIP-1β, RANTES and the like), endoserine, enterogastrin, histamine, neurotensin, TRH, pancreatic polypeptide or galanin.

Typically for performing a method for determining a ligand for a receptor protein of the invention or a salt thereof, a cell containing the receptor protein of the invention or a membrane fraction of the cell is suspended in a buffer suitable for the determination method to prepare a receptor standard. Such buffer may for example be a buffer which does not inhibit the binding between a ligand and the receptor protein, such as a phsophate buffer or tris-HCl buffer, pH 4 to 10 (preferably pH 6 to 8). Also for the purpose of reducing the non-specific binding, a surfactant such as CHAPS, Tween-80™ (KAO-ATLAS), digitonin, deoxycholate and the like or various proteins such as bovine serum albumin and gelatin may be added to a buffer. In addition, for the purpose of suppressing the cleavage of a receptor or a ligand by a protease, a protease inhibitor such as PMSF, leupeptine, E-64 (PEPTIDE KENKYUSHO) and a pepstatin may also be added. In a 0.01 ml to 10 ml of a solution of the receptor described above, a certain amount (5000 cpm to 500000 cpm) of $[^3H]$-, $[^{125}I]$-, $[^{14}C]$- or $[^{35}S]$-labeled test substance is contained. For measuring the non-specific binding (NSB) level, a reaction tube containing a large excess of the non-labeled test compound may also be provided. The reaction is performed at a temperature of about 0°C to 50°C, preferably about 4°C to 37°C for a period of about 20 minutes to 24 hours, preferably about 30 minutes to 3 hours. After the reaction, the mixture is filtered through a filter such as a glass fiber filter paper and washed with an appropriate volume of the same buffer and then the radioactivity remaining on the glass fiber filter paper is determined by a liquid scintillation counter or a gamma-counter. A test compound whose count (B-NSB) obtained by subtracting the non-specific binding (NSB) level from the total binding (B) level exceeded 0 cpm can be selected as a ligand (agonist) for the receptor protein of the invention or a salt thereof.

In order to perform the methods [4] to [5] described above for determining a ligand for a receptor protein of the invention or a salt thereof, such receptor protein-mediated cell stimulating activity (for example, an activity which promotes or suppresses arachidonic acid release, acetylcholin release, intracellular $Ca^{2+}$ release, intracellular cAMP production, intracellular cGMP production, inositol phosphate production, cell membrane potential variation, intracellular protein phosphorylation, c-fos activation, pH reduction and the like) can be determined by a known method or using a commercial assay kit. Typically, a cell containing a receptor protein is incubated in a multi-well plate. For determining a ligand, the medium is replaced with a fresh medium or an appropriate buffer which is non-toxic to the cell, and a test compound is added and incubated for a certain period, and then the cell is extracted or the supernatant is recovered and the product accumulated is quantified by a relevant method. When the production of a substance which is an index for a cell stimulating activity (for example, arachidonic acid) is difficult to be assayed due to any degrading enzyme contained in the cell, an inhibitor of such degrading enzyme may be added to perform the assay.

Another activity such as a cAMP production inhibiting activity can be detected as a production inhibiting effect in a cell whose basal production has been increased using forskolin and the like.

A kit of the invention for determining a ligand which binds to a receptor protein of the invention or a salt thereof comprises the receptor protein of the invention or a salt thereof, a partial peptide of the invention or a salt thereof, a cell containing the receptor protein of the invention or a membrane fraction of a cell containing the receptor protein of the invention.

A kit of the invention for determining a ligand may for example be one of those listed below.

1. Ligand determination reagents

[1] Assay buffer and washing buffer

Hanks' Balanced Salt Solution (Gibco) supplemented with 0.05 % bovine serum albumin (Sigma).

The buffer is sterilized by filtering through a filter whose pore size is 0.45 µm and stored at 4°C or prepared just before use.

[2] G-protein coupled receptor protein standard

A CHO cell expressing a receptor protein of the invention is subjected to a subculture in a 12-well plate at the density of 5 x $10^5$ cells/well and incubated at 37° C under 5% $CO_2$ and 95 % air for 2 days.

[3] Labeled test compound

A commercially available [$^3$H]-, [$^{125}$I]-, [$^{14}$C]- or [$^{35}$S]-labeled compound or an appropriately labeled compound.

A compound in an aqueous solution is stored at 4° C or -20°C and diluted at 1 µM with an assay buffer just before use. A water-insoluble test compound is dissolved in dimethylformamide, DMSO, methanol and the like.

[4] Non-labeled test compound

The compound identical to the labeled compound is prepared at a concentration higher by 100 to 1000 times.

2. Assay

[1] An inventive receptor protein-expressing CHO cell which has been incubated in a 12-well tissue culture plate is washed twice with 1 ml of an assay buffer and each 490 µl of the assay buffer is added to each well.

[2] 5 µl of a labeled test compound was added and allowed to react at room temperature for 1 hour. 5 µl of a non-labeled test compound is also added for measuring the non-specific binding.

[3] The reaction mixture is removed and the cell is washed three times with 1 ml of the washing buffer. The labeled test compound bound to the cell is dissolved by 0.2 N NaOH-1% SDS, and mixed with 4 ml of a liquid scintillator A (Wako Pure Chemical).

[4] Using a liquid scintillation counter (Beckman), the radioactivity is determined.

A ligand capable of being bound to a receptor protein of the invention or a salt thereof may for example be a substance existing specifically in brain, pituitary and pancreas, such as angiotensin, bonbesin, cannabinoid, cholecystokinin, glutamin, serotonin, melatonin, neuropeptide Y, opioid, purine, vasopressin, oxytocin, PACAP, secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene-related peptide), leucotriene, pancreastatin, prostaglandine, thromboxane, adenosine, adrenaline, α and β-chemokine (for example, IL-8, GROα, GROβ, GROγ, NAP-2, ENA-78, PF4, IP10, GCP-2, MCP-1, HC14, MCP-3, I-309, MIP1α, MIP-1β, RANTES and the like), endoserine, enterogastrin, histamine, neurotensin, TRH, pancreatic polypeptide, galanin and the like.

(2) Preventing and/or treating agent against disease related to dysfunction of G-protein coupled receptor protein of the invention

Once a ligand for a receptor protein of the invention has been characterized in the method (1) described above, it becomes possible to use [1] the receptor protein of the invention or [2] a DNA encoding this receptor protein as a pharmaceutical for preventing and/or treating a disease related to the dysfunction of the receptor protein of the invention in accordance with a function which the ligand has.

For example, in a patient whose ligand is not allowed to exert a physiological effect due to an in vivo reduction in a receptor protein of the invention (a patient having a deficiency of the receptor protein), the in vivo receptor protein in this patient can be increased to allow the ligand to exert the effect sufficiently by [1] administering the receptor protein of the invention to the patient to supplement the receptor protein, or by [2] (a) administering a DNA encoding the receptor protein of the invention to the patient to effect an expression of the protein or (b) inserting a DNA encoding the receptor protein of the invention into a target cell where the protein is expressed followed by implanting the cell into the patient. Thus, a DNA encoding a receptor protein of the invention is useful as a safe pharmaceutical low in toxicity for preventing and/or treating a disease related to the dysfunction of the receptor protein of the invention.

A receptor protein of the invention has a homology of about 30%, at the level of the amino acid sequence, with MAS which is one of G-protein coupled receptor proteins. Since it was reported that a change in the central nervous functions such as an increased anxiety was observed in an MAS gene defect mouse [J.B.C., 273 (No.19), 11867-11873 (1998)], the MAS gene is considered to play some role in the expression of the central nervous functions. Accordingly, a receptor protein of the invention having a homology with the MAS is useful in preventing and/or treating a disease related to an insufficiency of the central nervous functions (psychosis such as anxiety, schizophrenia, manic-depressive, dementia, retardation and dyskinesia). Also since it was reported that the expression of a rat MAS gene was increased in various peripheral organs just after delivery and then such high expression was still observed in the testis in addition to the central nervous system once after being matured [FEBS Lett. 357:27-32 (1995)], an important role in the cell growth, the acquisition of functions and the reproduction is also suggested. Accordingly, a receptor protein of the invention having a homology with the MAS is useful in preventing and/or treating respiratory, circulatory, digestive, hepatic/biliary/pancreatic and endocrinal diseases.

When a receptor protein of the invention is used as a preventing and/or treating agent as discussed above, a standard method can be employed to obtain a dosage form.

On the other hand, when a DNA encoding a receptor protein of the invention (hereinafter sometimes abbreviated as a DNA of the invention) is used as a preventing and/or treating agent described above, the DNA of the invention can be administered directly as it is or after an insertion into a suitable vector such as a retrovirus vector, an adenovirus vector, an adenovirus-associated virus vector and the like by a standard method. A DNA of the invention can be administered, as it is or in a formulation together with an auxiliary agent for promoting ingestion, using a gene gun or a catheter such as a hydrogel catheter.

For example, [1] a receptor protein of the invention or [2] a DNA encoding such receptor protein may be given orally as an optionally sugar-coated tablet, capsule, elixir, microcapsule and the like, or parenterally as a formulation for injection such as an aseptic solution or suspension in water or a pharmacologically acceptable liquid. For example, such formulation can be produced by mixing [1] a receptor protein of the invention or [2] a DNA encoding such receptor protein with a known physiologically acceptable carrier, flavor, excipient, vehicle, preservative stabilizer, binder and the like in a unit dosage form which is acceptable generally in pharmaceutical practice. The amount of an active ingredient in such formulation should be adjusted to achieve a suitable dose within a specified range.

An additive which may be incorporated into a tablet or a capsule may for example be a binder such as gelatin, corn starch, tragacanth, gum arabic and the like, an excipient such as crystalline cellulose, an expander such as corn starch, gelatin, alginic acid and the like; a lubricant such as magnesium stearate, a sweetener such as sucrose, lactose or saccharin, a flavor such as peppermint, oil of Geultheria ovatifolia spp., cherry and the like. When a unit dosage form is a capsule, a liquid carrier such as a fat may further be incorporated in addition to the materials described above. An aseptic formulation for injection can be prepared in accordance with ordinary pharmaceutical practice such as a dissolution or a suspension of an active ingredient, a naturally-occurring vegetable oil such as sesame oil and palm oil in a vehicle such as water for injection. An aqueous liquid for injection may for example be physiological saline, an isotonic solution containing glucose or other auxiliary agents (for example, D-sorbitol, D-mannitol, sodium chloride) and the like, which may be used in combination with a suitable solubilizer such as an alcohol (for example, ethanol), a polyalcohol (for example, propylene glycol, polyethylene glycol), a non-ionic surfactant (for example, polysorbate 80™, HCO-50). An oily liquid may for example be sesame oil and soybean oil, which may be used in combination with a solubilizer such as benzyl benzoate, benzyl alcohol and the like.

In addition, a preventing and/or treating agent described above may be supplemented also with a buffer agent (for example, phosphate buffer, sodium acetate buffer), a pain-ameliorating agent (for example, benzalkonium chloride, procaine hydrochloride), a stabilizer (for example, human serum albumin, polyethylene glycol), a preservative (for example, benzyl alcohol, phenol), an antioxidant. A formulation for injection thus prepared is then filled usually in a suitable ampoule.

Since a formulation thus obtained is safe and low in toxicity, it can be administered to a human or a mammalian animal ( for example, rat, rabbit, sheep, swine, cattle, cat, dog, monkey and the like).

While a receptor protein of the invention may be given at a dose which varies depending on the patient to be treated, the target organ, the condition of the patient and the administration route, it can be given orally to an adult (60 kg) usually at a daily dose of about 0.1 mg to 100 mg, preferably about 1.0 to 50 mg, more preferably about 1.0 to 20 mg. When it is given parenterally, the single dose may vary depending on the patient to be treated, the target organ, the condition of the patient and the administration route, and may for example be given as a formulation for injection to an adult (60 kg) usually at a daily dose of about 0.01 mg to 30 mg, preferably about 0.1 to 20 mg, more preferably about 0.1 to 10 mg, which is given advantageously by an intravenous injection. Also in other animals, a dose calculated for a 60 kg body weight may be administered.

While a DNA of the invention may be given at a dose which varies depending on the patient to be treated, the target organ, the condition of the patient and the administration route, it can be given orally to an adult (60 kg) usually at a daily dose of about 0.1 mg to 100 mg, preferably about 1.0 to 50 mg, more preferably about 1.0 to 20 mg. When it is given parenterally, the single dose may vary depending on the patient to be treated, the target organ, the condition of the patient and the administration route, and may for example be given as a formulation for injection to an adult (60 kg) usually at a daily dose of about 0.01 mg to 30 mg, preferably about 0.1 to 20 mg, more preferably about 0. 1 to 10 mg, which is given advantageously by an intravenous injection. Also in other animals, a dose calculated for a 60 kg body weight may be administered..

(3) Gene diagnostic agent

Since a DNA of the invention, when used as a probe, can detect an abnormality (gene abnormality) in a DNA or an mRNA encoding a receptor protein or its partial peptide of the invention in a human or a mammalian animal (for example, rat, rabbit, sheep, swine, cattle, cat, dog, monkey and the like), it is useful in a gene diagnosis of an impairment, a mutation or a reduced expression of such DNA or mRNA as well as an increase in or an increased expression of such DNA or mRNA.

A gene diagnosis employing a DNA of the invention can be performed for example by a northern hybridization known per se or a PCR-SSCP method (Genomics, Vol.5, p874-879 (1989)) or a method described in Proceedings of the National Academy of Sciences of USA, Vol.86, p2766-2770 (1989).

(4) Method for screening compound capable of altering expression level of receptor protein of the invention or a partial peptide thereof

A DNA of the invention, when used as a probe, can be applied to a screening for a compound which alters the expression level of a receptor protein of the invention or a partial peptide thereof.

Thus, the invention provides a method for screening for a compound which alters the expression level of a receptor protein of the invention or a partial peptide thereof, for example, by determining the level of an mRNA of the receptor protein of the invention or the partial peptide thereof contained in (i) [1] blood, [2] a certain organ and [3] a tissue or a cell isolated from an organ of a non-human mammal or in (ii) a transformant.

Typically, the level of an mRNA of a receptor protein of the invention or a partial peptide thereof is determined as follows.

(i) A normal or a disease-bearing non-human mammalian model animal (for example, mouse, rat, rabbit, sheep, swine, cattle, cat, dog, monkey and the like, more specifically, dementia rat, obesity mouse, arteriosclerosis rabbit, bile cancer mouse and the like) is treated with an agent (for example, anti-dementia agent, hypotensive agent, anti-cancer agent, anti-obesity agent and the like) or subjected to a mechanical stress (for example, flooding stress, electric shock, dark/light cycle, low temperature and the like), and after a certain time period, blood, a certain organ (for example, brain, liver, kidney and the like) or a tissue isolated from an organ or a cell is collected.

The mRNA contained in a receptor protein of the invention or a partial peptide thereof contained in a cell collected as described above can be quantified by extracting the mRNA from the cell by a standard method followed for example by TacManPCR, or can be analyzed by a northern blotting using a means known per se.

(ii) A transformant which expresses a receptor protein of the invention or a partial peptide thereof is prepared according to the method described above and a mRNA of the receptor protein of the invention or the partial peptide thereof contained in this transformant can be quantified and similarly analyzed.

A screening for a compound altering the expression level of a receptor protein of the invention or a partial peptide thereof can be accomplished by:

(i) administering to a normal or disease-bearing non-human mammalian model animal a test compound at a certain time point before (30 minutes to 24 hours, preferably 30 minutes to 12 hours, more preferably 1 hour to 6 hours before) or after (30 minutes to 3 days, preferably 1 hour to 2 days, more preferably 1 hour to 24 hours after) the exposure to a medication or a mechanical stress, or simultaneously with a medication or a mechanical stress, followed by quantifying and analyzing a mRNA of the receptor protein of the invention or the partial peptide thereof contained in a cell at a certain time point after (30 minutes to 3 days, preferably 1 hour to 2 days, more preferably 1 hour to 24 hours after) the administration;

(ii) adding a test compound to a culture medium in which a transformant-is incubated by a standard method followed by quantifying and analyzing a mRNA of the receptor protein of the invention or the partial peptide thereof contained in the transformant at a certain time point after (1 day to 7 days, preferably 1 day to 3 days, more preferably 2 days to 3 days after) initiation of the incubation.

A compound or a salt thereof obtained by a screening method of the invention is a compound having an ability of altering the expression level of a receptor protein of the invention or a partial peptide thereof, and typically it is (a) a compound which enhances a G-protein coupled receptor protein-mediated cell stimulating activity (for example, an activity which promotes or suppresses arachidonic acid release, acetylcholin release, intracellular $Ca^{2+}$ release, intracellular cAMP production, intracellular cGMP production, inositol phosphate production, cell membrane potential variation, intracellular protein phosphorylation, c-fos activation, pH reduction and the like) by increasing the expression level of a receptor protein of the invention or a partial peptide thereof, and (b) a compound which suppresses such cell stimulating activity by reducing the expression level of a receptor protein of the invention or a partial peptide thereof.

Such compound may for example be a peptide, a protein, a non-peptide compound, a synthetic compound, a fermented compound and the like, which may be novel compounds or known compounds.

A compound enhancing such cell stimulating activity is useful as a safe and pharmaceutical low in toxicity for enhancing a physiological activity of a receptor protein of the invention.

A compound suppressing such cell stimulating activity is useful as a safe and pharmaceutical low in toxicity for reducing the physiological activity of a receptor protein of the invention.

When a compound or a salt thereof obtained by a screening method of the invention is used in a pharmaceutical composition, a standard procedure may be followed. For example, similarly to a pharmaceutical containing a receptor protein of the invention, a dosage form such as tablet, capsule, elixir, microcapsule, aseptic solution or suspension can be formulated.

Since a formulation thus obtained is safe and low in toxicity, it can be administered to a human or a mammalian animal (for example, rat, rabbit, sheep, swine, cattle, cat, dog, monkey and the like).

While such compound may be given at a dose which varies depending on the patient to be treated, the target organ, the condition of the patient and the administration route, it can be given orally to an adult (60 kg) usually at a daily dose of about 0.1 mg to 100 mg, preferably about 1.0 to 50 mg, more preferably about 1.0 to 20 mg. When it is given parenterally, the single dose may vary depending on the patient to be treated, the target organ, the condition of the patient and the administration route, and may for example be given as a formulation for injection to an adult (60 kg) usually at a daily dose of about 0.01 mg to 30 mg, preferably about 0.1 to 20 mg, more preferably about 0.1 to 10 mg, which is given advantageously by an intravenous injection. Also in other animals, a dose calculated for a 60 kg body weight may be administered.

(5) Preventing and/or treating agent against various diseases containing compound capable of altering expression level of a receptor protein of the invention or a partial peptide thereof

As described above, a receptor protein of the invention is considered to play some important in vivo role for example in central nervous function. Accordingly, a compound altering the expression level of a receptor protein of the invention or a partial peptide thereof can be used as a preventing and/or treating agent against a disease related to the dysfunction of the receptor protein of the invention.

When such compound is used as a preventing and/or treating agent against a disease related to the dysfunction of a receptor protein of the invention, a standard formulation procedure may be followed.

For example, such compound may be given orally as an optionally sugar-coated tablet, capsule, elixir, microcapsule and the like, or parenterally as a formulation for injection such as an aseptic solution or suspension in water or pharmacologically acceptable liquid. For example, such formulation can be produced by mixing the compound with a known physiologically acceptable carrier, flavor, excipient, vehicle, preservative, stabilizer, binder and the like in a unit dosage form which is acceptable generally in pharmaceutical practice. The amount of an active ingredient in such formulation should be adjusted to achieve a suitable dose within a specified range.

An additive which may be incorporated into a tablet or a capsule may for example be a binder such as gelatin, corn starch, tragacanth, gum arabic and the like, an excipient such as crystalline cellulose, an expander such as corn starch, gelatin, alginic acid and the like, a lubricant such as magnesium stearate, a sweetener such as sucrose, lactose or saccharin, a flavor such as peppermint, oil of Geultheria ovatifolia spp., cherry and the like. When a unit dosage form is a capsule, a liquid carrier such as a fat may further be incorporated in addition to the materials described above. An aseptic formulation for injection can be prepared in accordance with ordinary pharmaceutical practice such as a dissolution or a suspension of an active ingredient, a naturally-occurring vegetable oil such as sesame oil and palm oil in a vehicle such as water for injection. An aqueous liquid for injection may for example be physiological saline, an isotonic solution containing glucose or other auxiliary agents (for example, D-sorbitol, D-mannitol, sodium chloride) and the like, which may be used in combination with a suitable solubilizer such as an alcohol (for example, ethanol), a polyalcohol (for example, propylene glycol, polyethylene glycol), a non-ionic surfactant (for example, polysorbate 80™, HCO-50) . An oily liquid may for example be sesame oil and soybean oil, which may be used in combination with a solubilizer such as benzyl benzoate, benzyl alcohol and the like.

In addition, a preventing and/or treating agent described above may be supplemented also with a buffer agent (for example, phosphate buffer, sodium acetate buffer), a pain-ameliorating agent (for example, benzalkonium chloride, procaine hydrochloride), a stabilizer (for example, human serum albumin, polyethylene glycol), a preservative (for example, benzyl alcohol, phenol), an antioxidant. A formulation for injection thus prepared is then filled usually in a suitable ampoule.

Since a formulation thus obtained is safe and low in toxicity, it can be administered to a human or a mammalian animal (for example, rat, rabbit, sheep, swine, cattle, cat, dog, monkey and the like).

While such compound or a salt there of may be given at a dose which varies depending on the patient to be treated, the target organ, the condition of the patient and the administration route, it can be given orally to an adult (60 kg) usually at a daily dose of about 0.1 mg to 100 mg, preferably about 1.0 to 50 mg, more preferably about 1.0 to 20 mg. When it is given parenterally, the single dose may vary depending on the patient to be treated, the target organ, the condition of the patient and the administration route, and may for example be given as a formulation for injection to an adult (60 kg) usually at a daily dose of about 0.01 mg to 30 mg, preferably about 0.1 to 20 mg, more preferably about 0.1 to 10 mg, which is given advantageously by an intravenous injection. Also in other animals, a dose calculated for a 60 kg body weight may be administered.

(6) Method for quantifying ligand for G-protein coupled receptor protein of the invention

Since a receptor protein of the invention has a binding affinity to a ligand, it can quantify an in vivo ligand concentration at a high sensitivity.

A quantification method of the invention can be employed for example in combination with a competitive method. Thus, a sample is brought into contact with a receptor of the invention and the like to determine the concentration of a ligand in the sample. Typically, a method described in literatures [1] and [2] shown below or an analogous method may be employed.

[1] Ed. by H. Irie, "Radioimmunoassay", (KODANSHA, 1974)
[2] Ed. by H. Irie, "Radioimmunoassay, 2nd Vol.", (KODANSHA, 1979)

(7) Method for screening a compound (agonist, antagonist) capable of altering binding affinity between G-protein coupled receptor protein of the invention and ligand

By using a receptor protein and the like of the invention, or by constructing an expression system of a recombinant receptor protein followed by employing a receptor binding assay system employing this expression system, a compound altering the binding affinity between a ligand and the receptor protein of the invention and the like (for example, peptide, protein, non-peptide compound, synthetic compound, fermented compound and the like) a salt thereof can efficiently be screened for.

Such compound includes (a) a compound having a G-protein coupled receptor-mediated cell stimulating activity (for example, an activity which promotes or suppresses arachidonic acid release, acetylcholin release, intracellular $Ca^{2+}$ release, intracellular cAMP production, intracellular cGMP production, inositol phosphate production, cell membrane potential variation, intracellular protein phosphorylation, c-fos activation, pH reduction and the like) (so-called agonist for a receptor protein of the invention), (b) a compound having no such cell stimulating activity (so-called antagonist for a receptor protein of the invention), (c) a compound enhancing the binding affinity between a ligand and a G-protein coupled receptor protein of the invention, or (d) a compound reducing the binding affinity between a ligand and a G-protein coupled receptor protein of the invention (it is preferred to screen for a compound defined as (a) by a ligand determination method described above).

Thus, the invention provides a method for screening a compound or a salt thereof capable of altering the binding affinity between a ligand and a receptor protein of the invention or a partial peptide or a salt thereof comprising a comparison between (i) a contact of a receptor protein of the invention or a partial peptide thereof or a salt thereof with the ligand and (ii) a contact of the receptor protein of the invention or the partial peptide thereof or a salt thereof with the ligand and a test compound.

A screening method of the invention is characterized by a determination of and a comparison between the levels of the binding of a ligand to such receptor protein and the cell stimulating activities upon a case (i) and upon a case (ii).

More typically, the invention provides:

[1] a method for screening a compound or a salt thereof capable of altering the binding affinity between a ligand and a receptor protein and the like of the invention comprising a determination of and a comparison between the levels of the binding of a labeled ligand to the receptor protein of the invention and the like upon a contact of the labeled ligand with the receptor protein of the invention and the like and upon a contact of the labeled ligand and a test compound with the receptor protein of the invention and the like;

[2] a method for screening a compound or a salt thereof capable of altering the binding affinity between a ligand and a receptor protein and the like of the invention comprising a determination of and a comparison between the levels of the binding of a labeled ligand to a cell or a membrane fraction thereof comprising the receptor protein of the invention and the like upon a contact of the labeled ligand with said cell or the membrane fraction thereof and upon a contact of the labeled ligand and a test substance with said cell or the membrane fraction thereof;

[3] a method for screening a compound or a salt thereof capable of altering the binding affinity between a ligand and a receptor protein and the like of the invention comprising a determination of and a comparison between the levels of the binding of a labeled ligand to the receptor protein and the like expressed on the cell membrane by incubating a transformant containing a DNA of the invention upon a contact of the labeled ligand with such receptor protein and the like and upon a contact of the labeled ligand and a test substance with such receptor protein;

[4] a method for screening a compound or a salt thereof capable of altering the binding affinity between a ligand and a receptor protein and the like of the invention comprising a determination of and a comparison between a receptor-mediated cell stimulating activities (for example, an activity which promotes or suppresses arachidonic acid release, acetylcholin release, intracellular $Ca^{2+}$ release, intracellular cAMP production, intracellular cGMP production, inositol phosphate production, cell membrane potential variation, intracellular protein phosphorylation, c-fos activation, pH reduction and the like) upon a contact of a compound activating the receptor protein and the like (for example, a ligand for the receptor protein of the invention) with a cell comprising the receptor protein and the like and upon a contact of a compound activating the receptor protein and the like and a test compound with the cell comprising the receptor protein and the like; and,

[5] a method for screening a compound or a salt thereof capable of altering the binding affinity between a ligand and a receptor protein and the like of the invention comprising a determination of and a comparison between a receptor-mediated cell stimulating activity (for example, an activity which promotes or suppresses arachidonic acid release, acetylcholin release, intracellular $Ca^{2+}$ release, intracellular cAMP production, intracellular cGMP production, inositol phosphate production, cell membrane potential variation, intracellular protein phosphorylation, c-fos activation, pH reduction and the like) upon a contact of a compound activating the receptor protein and the like (for example, a ligand for the receptor protein of the invention) with the receptor protein and the like expressed on the cell membrane by incubating a transformant containing a DNA of the invention and upon a contact of the compound activating the receptor protein and the like and a test compound with the receptor protein and the like expressed on the cell membrane by incubating a transformant containing a DNA of the invention.

In the days when a receptor protein and the like of the invention was not available, screening for a G-protein coupled receptor agonist or antagonist involved the use of a G-protein coupled receptor protein-containing cell, tissue or cell membrane fraction, for example, of a rat to obtain a candidate compound (primary screening) followed by a test for verifying that the candidate compound surely inhibits the binding between a human G-protein coupled receptor protein and a ligand (secondary screening). Since other receptor proteins were allowed to be present simultaneously when such cell, tissue or cell membrane was used as it is, it was difficult practically to screen for an agonist or antagonist for an intended receptor protein.

On the contrary, the use of a human-derived receptor protein obtained according to the invention requires no such primary screening and allows a compound which inhibits the binding between a ligand and a G-protein coupled receptor protein to be selected for at a high efficiency. In addition, a convenient judgement whether a selected compound is an agonist or an antagonist becomes possible.

A typical screening method of the invention is described below.

A receptor protein and the like of the invention employed in a screening method of the invention may be any one containing a receptor protein of the invention and the like described above, and is preferably a cell membrane fraction of an organ of a mammalian animal containing the receptor protein of the invention and the like. Nevertheless, a screening may employ a human-derived receptor protein which is expressed in a large amount using a recombinant since an organ derived especially from a human is extremely difficult to obtain.

In order to produce a recept or protein of the invention, an expression method described above may be employed, and it is preferred to express a DNA of the invention in a mammalian cell or an insect cell. While a complementary DNA is employed as a DNA fragment encoding a target protein moiety, it is not limiting. For example, a gene fragment or a synthetic DNA may also be employed. For the purpose of introducing a DNA fragment encoding a receptor protein of the invention into an animal host cell to effect a highly efficient expression, it is preferred to integrate such DNA fragment downstream of a polyhedron promoter of a nuclear polyhedrosis virus (NPV) classified as a baculovirus whose host is an insect, an SV 40-derived promoter, a retrovirus promoter, a metallothioneine promoter, a human heat shock promoter, a cytomegalovirus promoter, an SRα promoter and the like. An expressed receptor can be examined for its quantity or quality by a method known per se. For example, a method known in the literature (Nambi, P. et al., J.Biol. Chem., Vo.267, p.19555 to 19559, 1992) may be employed.

Accordingly, in a screening method of the invention, those containing a receptor protein and the like of the invention may be a receptor protein and the like which was purified by a method known per se or a cell containing such receptor protein or a cell membrane fraction containing such receptor protein.

When a cell containing a receptor protein of the invention is employed in a screening method of the invention, this cell may be immobilized by glutaraldehyde or formalin. Such immobilization can be effected by a method known per se.

A cell containing a receptor protein of the invention is a host cell expressing the receptor protein of the invention, and such host cell may preferably be an Escherichia microorganism, a Bacillus microorganism, a yeast, an insect cell, an animal cell and the like.

A cell membrane fraction means a cell membrane-rich fraction obtained by a method known per se after pelletizing the cell. A cell may be pelletized for example by a method in which a cell is pressed and crushed by a Potter-Elvehjem homogenizer, by using a whirling blender or a polytron (Kinematica), by means of an ultrasonic treatment, or by a method in which a cell is sprayed via a fine nozzle while being pressurized by a French press. A cell membrane may be fractionated mainly by a centrifugal fractionation such as a fractional centrifugation or a density gradient centrifugation. For example, a cell pellet is centrifuged at a low speed (500 rpm to 3000 rpm) for a short period (usually about 1 minutes to 10 minutes) to obtain a supernatant, which is then centrifuged at a higher speed (15000 rpm to 30000 rpm) usually for 30 minutes to 2 hours to obtain a pellet, which is used as a membrane fraction. This membrane fraction contains a large amount of the membrane components such as an expressed receptor protein and phospholipids and membrane proteins derived from the cell.

The amount of a receptor protein in a cell containing such receptor protein or a membrane fraction thereof is preferably $10^3$ to $10^8$ molecules per cell, more preferably $10^5$ to $10^7$ molecules. A higher expression leads to a higher ligand binding activity (specific activity) per membrane fraction, whereby allowing not only a highly sensitive screening system to be established but also a large amount of a sample to be determined in an identical lot.

For performing the methods (1) to (3) described above for screening for a compound altering the binding affinity between a ligand and a receptor protein and the like of the invention, a suitable receptor protein fraction and a labeled test compound are required.

A receptor protein fraction is preferably a natural receptor protein fraction or a recombinant receptor protein fraction having an activity which is equivalent to that of the natural one. The expression "activity which is equivalent" employed here means an equivalent ligand binding activity or signal data transmission activity.

A labeled ligand may be a labeled ligand and a labeled ligand analogue compound. For example, a ligand labeled with [$^3$H], [$^{125}$I], [$^{14}$C] or [$^{35}$S] may be employed.

Typically for performing a method for screening for a compound altering the binding affinity between a ligand and a receptor protein and the like of the invention, a cell containing the receptor protein of the invention or a membrane fraction of the cell is suspended in a buffer suitable for the screening to prepare a receptor protein standard. Such buffer may for example be a buffer which does not inhibit the binding between a ligand and the receptor protein, such as a phsophate buffer or tris-HCl buffer, pH 4 to 10 (preferably pH 6 to 8). Also for the purpose of reducing the non-specific binding, a surfactant such as CHAPS, Tween-80™ (KAO-ATLAS) , digitonin, deoxycholate and the like may be added to a buffer. In addition, for the purpose of suppressing the cleavage of a receptor or a ligand by a protease, a protease inhibitor such as PMSF, leupeptine, E-64 (PEPTIDE KENKYUSHO) and a pepstatin may also be added. In a 0.01 ml to 10 ml of a solution of the receptor described above, a certain amount (5000 cpm to 500000 cpm) of a labeled ligand is added and a test compound is allowed to be present simultaneously at a concentration of $10^{-4}$M to $10^{-10}$M. For measuring the non-specific binding (NSB) level, a reaction tube containing a large excess of the non-labeled ligand may also be provided. The reaction is performed at a temperature of about 0°C to 50°C, preferably about 4°C to 37°C for a period of about 20 minutes to 24 hours, preferably about 30 minutes to 3 hours. After the reaction, the mixture is filtered through a filter such as a glass fiber filter paper and washed with an appropriate volume of the same buffer and then the radioactivity remaining on the glass fiber filter paper is determined by a liquid scintillation counter or a gamma-counter. By regarding the count ($B_0$-NSB) obtained by subtracting the non-specific binding (NSB) level from the binding ($B_0$) in the absence of any competitive substance as 100 %, a test compound whose specific binding (B-NSB) is 50 % or less can be selected as a candidate having a competitive inhibitory effect.

In order to perform the methods [4] to [5] described above for screening for a compound altering the binding affinity between a ligand and a receptor protein and the like of the invention, such receptor protein-mediated cell stimulating activity (for example, an activity which promotes or suppresses arachidonic acid release, acetylcholin release, intracellular $Ca^{2+}$ release, intracellular cAMP production, intracellular cGMP production, inositol phosphate production, cell membrane potential variation, intracellular protein phosphorylation, c-fos activation, pH reduction and the like) can be determined by a known method or using a commercial assay kit.

Typically, a cell containing a receptor protein and the like of the invention is incubated in a multi-well plate. For a screening, the medium is replaced with a fresh medium or an appropriate buffer which is non-toxic to the cell, and a test compound is added and incubated for a certain period, and then the cell is extracted or the supernatant is recovered and the product accumulated is quantified by a relevant method. When the production of a substance which is an index for a cell stimulating activity (for example, arachidonic acid) is difficult to be assayed due to any degrading enzyme contained in the cell, an inhibitor of such degrading enzyme may be added to perform the assay. Another activity such as a cAMP production inhibiting activity can be detected as a production inhibiting effect in a cell whose basal production has been increased using forskolin and the like.

To perform a screening with determining a cell stimulating activity, a suitable cell expressing a receptor protein is required. A cell expressing a receptor protein and the like of the invention is preferably a cell line having a natural receptor protein and the like of the invention and a cell line expressing a recombinant receptor protein and the like described above.

A test compound may be a peptide, a protein, a non-peptide compound, a synthetic compound, a fermented compound, a cell extract, a plant extract, an animal tissue extract and the like, which may be a novel compound or a known compound.

A kit of the invention for screening a compound or a salt thereof altering the binding affinity between a ligand and a receptor protein and the like of the invention comprises the receptor protein and the like of the invention, a cell containing the receptor protein and the like of the invention or a membrane fraction of a cell containing the receptor protein and the like of the invention.

A screening kit of the invention may for example be one of those listed below.

1. Screening reagents

[1] Assay buffer and washing buffer

Hanks' Balanced Salt Solution (Gibco) supplemented with 0.05 % bovine serum albumin (Sigma).
The buffer is sterilized by filtering through a filter whose pore size is 0.45 μm and stored at 4°C or prepared just before use.

[2] G-protein coupled receptor standard

A CHO cell expressing a receptor protein of the invention is subjected to a subculture in a 12-well plate at the density of $5 \times 10^5$ cells/well and incubated at 37° C under 5% $CO_2$ and 95 % air for 2 days.

[3] Labeled test compound

A commercially available [$^3$H]-, [$^{125}$I]-, [$^{14}$C]- or [$^{35}$S]-labeled ligand.
A ligand in an aqueous solution is stored at 4°C or -20°C and diluted at 1 μM with an assay buffer just before use.

[4] Ligand standard solution

A ligand is dissolved at 1 mM with a PBS supplemented with 0.1 % bovine serum albumin (Sigma) and stored at -20° C.

2. Assay

[1] An inventive receptor protein-expressing CHO cell which has been incubated in a 12-well tissue culture plate is washed twice with 1 ml of an assay buffer and each 490 μl of the assay buffer is added to each well.
[2] After adding 5 μl of a $10^{-3}$ to $10^{-10}$ M solution of a test compound, 5 μl of a labeled ligand is added and allowed to react at room temperature for 1 hour. 5 μl of a $10^{-3}$ M ligand is added instead of the test compound for measuring the non-specific binding.

[3] The reaction mixture is removed and the cell is washed three times with 1 ml of the washing buffer. The labeled ligand bound to the cell is dissolved by 0.2 N NaOH-1% SDS, and mixed with 4 ml of a liquid scintillator A (Wako Pure Chemical).

[4] Using a liquid scintillation counter (Beckman), the radioactivity is determined, and a percent maximum binding (PMB) is calculated as follows.

$$PMB= [(B-NSB) / (B_0-NSB)] \times 100$$

wherein PMB is a percent maximum binding, B is a binding level in the presence of a sample, NSB is a non-specific binding level and $B_0$ is the maximum binding level.

A compound or a salt obtained by a screening method or using a screening kit of the invention is a compound having an ability of altering the binding affinity between a ligand and a receptor protein and the like of the invention, and typically it is (a) a compound having a G-protein coupled receptor-mediated cell stimulating activity (for example, an activity which promotes or suppresses arachidonic acid release, acetylcholin release, intracellular $Ca^{2+}$ release, intracellular cAMP production, intracellular cGMP production, inositol phosphate production, cell membrane potential variation, intracellular protein phosphorylation, c-fos activation, pH reduction and the like) (so-called agonist for a receptor protein of the invention), (b) a compound having no such cell stimulating activity (so-called antagonist for a receptor protein of the invention), (c) a compound enhancing the binding affinity between a ligand and a G-protein coupled receptor protein of the invention, or (d) a compound reducing the binding affinity between a ligand and a G-protein coupled receptor protein of the invention.

Such compound may for example be a peptide, a protein, a non-peptide compound, a synthetic compound, a fermented compound and the like, which may be novel compounds or known compounds.

Since an agonist for a receptor protein and the like of the invention has a physiological effect similar to that possessed by a ligand for the receptor protein and the like of the invention, it is useful as a safe and pharmaceutical low in toxicity utilizing the relevant ligand activity.

Since an antagonist for a receptor protein and the like of the invention has an inhibitory effect on the physiological effect of a ligand for the receptor protein and the like of the invention, it is useful as a safe and pharmaceutical low in toxicity inhibiting the ligand activity.

A compound enhancing the binding affinity between a ligand and a G-protein coupled receptor protein of the invention is useful as a safe and pharmaceutical low in toxicity for enhancing the bioactivity of a ligand for the receptor protein and the like of the invention.

A compound reducing the binding affinity between a ligand and a G-protein coupled receptor protein of the invention is useful as a safe and pharmaceutical low in toxicity for reducing the bioactivity of a ligand for the receptor protein and the like of the invention.

When a compound or a salt thereof obtained by a screening method or using a screening kit of the invention is used in a pharmaceutical composition, a standard procedure may be followed. For example, similarly to a pharmaceutical containing a receptor protein of the invention, a dosage form such as tablet, capsule, elixir, microcapsule, aseptic solution or suspension can be formulated.

Since a formulation thus obtained is safe and low in toxicity, it can be administered to a human or a mammalian animal (for example, rat, rabbit, sheep, swine, cattle, cat, dog, monkey and the like).

While such compound may be given at a dose which varies depending on the patient to be treated, the target organ, the condition of the patient and the administration route, it can be given orally to an adult (60 kg) usually at a daily dose of about 0.1 mg to 100 mg, preferably about 1.0 to 50 mg, more preferably about 1.0 to 20 mg. When it is given parenterally, the single dose may vary depending on the patient to be treated, the target organ, the condition of the patient and the administration route, and may for example be given as a formulation for injection to an adult (60 kg) usually at a daily dose of about 0.01 mg to 30 mg, preferably about 0.1 to 20 mg, more preferably about 0.1 to 10 mg, which is given advantageously by an intravenous injection. Also in other animals, a dose calculated for a 60 kg body weight may be administered.

(8) Preventing and/or treating agent against various disease containing compounds (agonist, antagonist) capable of altering the binding affinity between G-protein coupled receptor protein of the invention and ligand

As described above, a receptor protein of the invention is considered to play some important in vivo role for example in central nervous function. Accordingly, a compound (agonist, antagonist) capable of altering the binding affinity between a receptor protein of the invention and a ligand can be used as a preventing and/or treating agent against a disease related to the dysfunction of the receptor protein of the invention.

When such compound is used as a preventing and/or treating agent against a disease related to the dysfunction of a receptor protein of the invention, a standard procedure may be followed.

For example, such compound may be given orally as an optionally sugar-coated tablet, capsule, elixir, microcapsule and the like, or parenterally as a formulation: for injection such as an aseptic solution or suspension in water or pharmacologically acceptable liquid. For example, such formulation can be produced by mixing the compound with a known physiologically acceptable carrier, flavor, excipient, vehicle, preservative, stabilizer, binder and the like in a unit dosage form which is acceptable generally in pharmaceutical practice. The amount of an active ingredient in such formulation should be adjusted to achieve a suitable dose within a specified range.

An additive which may be incorporated into a tablet or a capsule may for example be a binder such as gelatin, corn starch, tragacanth, gum arabic and the like, an excipient such as crystalline cellulose, an expander such as corn starch, gelatin, alginic acid and the like, a lubricant such as magnesium stearate, a sweetener such as sucrose, lactose or saccharin, a flavor such as peppermint, oil of Geultheria ovatifolia spp., cherry and the like. When a unit dosage form is a capsule, a liquid carrier such as a fat may further be incorporated in addition to the materials described above. An aseptic formulation for injection can be prepared in accordance with ordinary pharmaceutical practice such as a dissolution or a suspension of an active ingredient, a naturally-occurring vegetable oil such as sesame oil and palm oil in a vehicle such as water for injection. An aqueous liquid for injection may for example be physiological saline, an isotonic solution containing glucose or other auxiliary agents (for example, D-sorbitol, D-mannitol, sodium chloride) and the like, which may be used in combination with a suitable solubilizer such as an alcohol (for example, ethanol), a polyalcohol (for example, propylene glycol, polyethylene glycol), a non-ionic surfactant (for example, polysorbate 80™, HCO-50). An oily liquid may for example be sesame oil and soybean oil, which may be used in combination with a solubilizer such as benzyl benzoate, benzyl alcohol and the like.

In addition, a preventing and/or treating agent described above may be supplemented also with a buffer agent (for example, phosphate buffer, sodium acetate buffer), a pain-ameliorating agent (for example, benzalkonium chloride, procaine hydrochloride), a stabilizer (for example, human serum albumin, polyethylene glycol), a preservative (for example, benzyl alcohol, phenol), an antioxidant. A formulation for injection thus prepared is then filled usually in a suitable ampoule.

Since a formulation thus obtained is safe and low in toxicity, it can be administered to a human or a mammalian animal (for example, rat, rabbit, sheep, swine, cattle, cat, dog, monkey and the like).

While such compound or a salt thereof may be given at a dose which varies depending on the patient to be treated, the target organ, the condition of the patient and the administration route, it can be given orally to an adult (60 kg) usually at a daily dose of about 0.1 mg to 100 mg, preferably about 1.0 to 50 mg, more preferably about 1.0 to 20 mg. When it is given parenterally, the single dose may vary depending on the patient to be treated, the target organ, the condition of the patient and the administration route, and may for example be given as a formulation for injection to an adult (60 kg) usually at a daily dose of about 0.01 mg to 30 mg, preferably about 0.1 to 20 mg, more preferably about 0.1 to 10 mg, which is given advantageously by an intravenous injection. Also in other animals, a dose calculated for a 60 kg body weight may be administered.

(9) Quantification of receptor protein of the invention or partial peptide thereof or salt thereof

Since an antibody of the invention can recognize the receptor protein and the like of the invention specifically, it can be used for quantifying the receptor protein and the like of the invention in a test sample, especially by a sandwich immunoassay. Thus, the present invention also provides, for example, (i) a method for quantifying a receptor protein and the like of the invention in a test sample comprising a competitive reaction of an antibody of the invention with the test sample and a labeled receptor protein and the like of the invention followed by a determination of the ratio of the labeled receptor protein and the like of the invention bound to the antibody, and (ii) a method for quantifying a receptor protein and the like of the invention in a test sample comprising a simultaneous or sequential reaction of the test sample with an antibody of the invention insolubilized on a carrier and another labeled antibody of the invention followed by'a determination of the activity of the label on the insolubilized carrier.

In the method of (ii) described above, it is preferred that one antibody is an antibody recognizing the N-terminal of a receptor protein and the like of the invention and the other is reactive with the C-terminal of the receptor protein of the invention.

Using a monoclonal antibody against a receptor protein and the like of the invention (hereinafter sometimes referred to as a monoclonal antibody of the invention), the receptor protein and the like of the invention can be quantified and detection by a tissue staining and the like can also be performed. For these purposes, an antibody molecule itself may be used, or a F(ab')$_2$, Fab' or Fab fraction of the antibody molecule may also be employed. A method for quantifying a receptor protein and the like of the invention using an antibody of the invention is not particularly limited, and may be any method in which the amount of an antibody, antigen or antibody-antigen complex corresponding to the amount of the antigen (for example, the amount of the receptor protein) in a test sample is detected physically and then a calculation is made based on a standard curve obtained by using the standard solutions containing known amounts of the antigen. For example, a nephrometry, a competitive assay, an immunometric assay and a sandwich assay can preferably be employed, with a sandwich assay described below being preferred in view of the sensitivity and the specificity.

A label employed in an assay using a labeled substance may for example be a radioisotope, an enzyme, a fluorescent substance, a luminescent substance and the like. Such radioisotope may for example be [$^{125}$I], [$^{131}$I], [$^{3}$H], [$^{14}$C] and the like. An enzyme described above is preferably one which is stable and has a high specific activity and may for example be β-galactosidase, β-glucosidase, alkaline phosphatase, peroxidase, malate dehydrogenase and the like. A fluorescent substance may for example be *FLUORESCAMINE*, fluorescein isothiocyanate and the like. A luminescent substance may for example be luminol, a luminol derivative, luciferin, lucigenin and the like. A biotin-avidin system may also be employed for binding a label to an antibody or an antigen.

An insolubilization of an antigen or an antibody may be effected utilizing a physical adsorption, and a chemical binding employed usually for insolubilizing a protein or an enzyme may also be employed. A carrier may for example be an insoluble polysaccharide such as agarose, dextran and cellulose, a synthetic resin such as polystyrene, polyacrylamide and silicon, as well as a glass.

In a sandwich method, an insolubilized inventive monoclonal antibody is reacted with a test sample (primary reaction), and then another labeled monoclonal antibody of the invention is reacted (secondary reaction), and then the activity of the label on the insolubilized carrier is determined, whereby quantifying a receptor protein of the invention in the test sample. The primary reaction and the secondary reaction can be performed in the opposite order or may be performed simultaneously or at an interval. A label and a method for the insolubilization are as described above.

It is not always necessary in a sandwich immunoassay that an antibody employed as an antibody for a solid phase or an antibody for labeling is of a single type, and several antibodies can be employed as in a mixture for the purpose of a higher sensitivity of the measurement.

In a method for determining a receptor protein and the like by a sandwich method of the invention, monoclonal antibodies employed in the primary reaction and the secondary reaction are preferably those differing from each other in the site of the binding to the receptor protein and the like. Thus, the antibodies employed in the primary and secondary reactions are selected so that when the antibody employed in the secondary reaction recognizes the C-terminal of the receptor protein then the antibody employed in the primary reaction recognizes the sites other than the C-terminal, such as the N-terminal.

A monoclonal antibody can be used in an assay system other than a sandwich assay, such as a competitive assay, an immunometric assay and a nephrometry. In a competitive assay, an antigen in a test sample and a labeled antigen are reacted competitively with an antibody and then unreacted labeled antigen (F) is separated from an antibody-binding labeled antigen (B) (B/F separation), and the amount of the label on either B or F is determined, whereby quantifying the antigen in the test sample. This reaction is conducted by a liquid phase method employing a soluble antibody as an antibody and performing a B/F separation using a polyethylene glycol and a secondary antibody, and also by a solid phase method employing a solid phase antibody as a primary antibody or employing a soluble antibody as a primary antibody and a solid phase antibody as a secondary antibody.

In an immunometric method, an antigen in a test sample and a solid phase antigen are reacted competitively with a certain amount of a labeled antibody, and then the solid phase is separated from a liquid phase, or an antigen in a test sample is reacted with a labeled antibody in excess and then a solid phase antigen is added to bind an unreacted labeled antibody to the solid phase and subsequently the solid phase is separated from a liquid phase. Then the amount of the label in either phase is determined, whereby quantifying the antigen in the test sample.

In a nephrometric assay, the amount of an insoluble precipitate formed as a result of an antigen-antibody reaction in a gel or a solution is determined. Even when the amount of an antigen in a test sample is very small and only a small amount of a precipitation can be obtained, a laser nephrometry utilizing a scattering of the laser is preferably employed.

When applying each immunological assay described above to a quantification method of the invention, no particular condition or operation is specified. Ordinary conditions and operations in each method may be employed in combination with a technology known by those skilled in the art to construct an assay system for a receptor protein of the invention or a salt thereof. Such general technology is found in corresponding textbooks or guidebooks [for example, "Radioimmunoassay", ed. by H.Irie (KODANSHA, 1974), "Radioimmunoassay II", ed. by H.Irie (KODANSHA, 1979), "Enzyme Immunoassay", ed. by E. Ishikawa et al., (IGAKUSHOIN, 1978), "Enzyme Immunoassay", (2nd Volume), ed. by E. Ishikawa et al., (IGAKUSHOIN, 1982), "Enzyme Immunoassay", (3rd Volume), ed. by E.Ishikawa et al., (IGAKUSHOIN, 1987), "Methods in Enzymology", Vol.70, (Immunochemical Techniques (Part A)), Vol.73, (Immunochemical Techniques (Part B)), Vol.74, (Immunochemical Techniques (Part C)), Vol.84, (Immunochemical Techniques (Part D: Selected Immunoassays)), Vol.92, (Immunochemical Techniques (Part E: Monoclonal Antibodies and General Immunoassay Methods)), Vol.121, (Immunochemical Techniques (Part I: Hybridoma Technology and Monoclonal Antibodies)), (Academic Press)].

As described above, a receptor protein of the invention or a salt thereof can be quantified at a high sensitivity by employing an antibody of the invention.

Furthermore, by quantifying a receptor protein of the invention or a salt thereof in vivo using an antibody of the invention, various diseases related to the dysfunction of the receptor protein of the invention can be diagnosed.

An antibody of the invention can also be employed for a specific detection of a receptor protein and the like of the invention which is present in a test sample such as a body fluid or a tissue. It can also be employed in preparing an antibody column used for purifying a receptor protein and the like of the invention, in detecting a receptor protein and the like of the invention in each fraction upon a purification, or in analyzing the behavior of a receptor protein and the like of the invention in a test cell.

(10) Method for screening compound capable of altering amount of receptor protein of the invention or partial peptide thereof in cell membrane

Since an antibody of the invention can specifically recognize a receptor protein or a partial peptide or a salt thereof, it can be used for screening for a compound altering the amount of the receptor protein or the partial peptide or the salt of the invention in a cell membrane.

Thus, the present invention provides, for example:

(i) a method for screening for a compound altering the amount of a receptor protein of the invention or a partial peptide thereof in a cell membrane by destroying [1] blood, [2] a certain organ or [3] a tissue or a cell isolated from an organ of a non-human mammalian animal followed by isolating a cellmembrane fraction followed by quantifying the receptor protein of the invention or the partial peptide thereof contained in the cell membrane fraction;

(ii) a method for screening for a compound altering the amount of a receptor protein of the invention or a partial peptide thereof in a cell membrane by destroying a transformant expressing the receptor protein of the invention or the partial peptide thereof followed by isolating a cell membrane fraction followed by quantifying the receptor protein of the invention or the partial peptide thereof contained in the cell membrane fraction;

(iii) a method for screening for a compound altering the amount of a receptor protein of the invention or a partial peptide thereof in a cell membrane by cutting [1] blood, [2] a certain organ or [3] a tissue or a cell isolated from an organ of a non-human mammalian animal into sections followed by using an imminostaining method to quantify the staining of the receptor protein in the surface layer of the cell whereby identifying the protein on the cell membrane. Also provided is (iv) a method for screening for a compound altering the amount of a receptor protein of the invention or a partial peptide thereof in a cell membrane by cutting a transformant expressing the receptor protein of the invention or the partial peptide thereof into sections followed by using an imminostaining method to quantify the staining of the receptor protein in the surface layer of the cell whereby identifying the protein on the cell membrane.

Typically, the level of a receptor protein of the invention or a partial peptide thereof contained in a cell membrane fraction is quantified as follows.

(i) A normal or a disease-bearing non-human mammalian model animal (for example, mouse, rat, rabbit, sheep, swine, cattle, cat, dog, monkey and the like, more specifically, dementia rat, obesity mouse, arteriosclerosis rabbit, bile cancer mouse and the like) is treated with an agent (for example, anti-dementia agent, hypotensive agent, anti-cancer agent, anti-obesity agent and the like) or subjected to a mechanical stress (for example, flooding stress, electric shock, dark/light cycle, low temperature and the like), and after a certain time period, blood, a certain organ (for example, brain, liver, kidney and the like) or a tissue or a cell isolated from an organ is collected. The organ, tissue or cell thus obtained is suspended in a suitable buffer solution (for example, Tris-HCl buffer, phosphate buffer, Hepes buffer) where it is destroyed and then exposed to a surfactant (for example Triton X100™, Tween 20™) and then subjected to a centrifugation, a filtration or a column fractionation, whereby obtaining a cell membrane fraction.

A cell membrane fraction means a cell membrane-rich fraction obtained by a method known per se after pelletizing the cell. A cell may be pelletized for example by a method in which a cell is pressed and crushed by a Potter-Elvehjem homogenizer, by using a whirling blender or a polytron (Kinematica), by means of an ultrasonic treatment, or by a method in which a cell is sprayed via a fine nozzle while being pressurized by a French press. A cell membrane may be fractionated mainly by a centrifugal fractionation such as a fractional centrifugation or a density gradient centrifugation. For example, a cell pellet is centrifuged at a low speed (500 rpm to 3000 rpm) for a short period (usually about 1 minutes to 10 minutes) to obtain a supernatant, which is then centrifuged at a higher speed (15000 rpm to 30000 rpm) usually for 30 minutes to 2 hours to obtain a pellet, which is used as a membrane fraction. This membrane fraction contains a large amount of the membrane components such as an expressed receptor protein and phospholipids and membrane proteins derived from the cell.

A receptor protein or a partial peptide of the invention contained in a cell membrane fraction can be quantified for example by a sandwich immunoassay employing an antibody of the invention as well as western blotting.

Such sandwich immunoassay can be performed similarly to a method described above, while the western blotting can be performed by a method known per se.

(ii) A transformant which expresses a receptor protein of the invention or a partial peptide thereof is prepared according to the method described above and the receptor protein of the invention or the partial peptide thereof contained in a cell membrane fraction can be quantified and analyzed similarly.

A screening for a compound altering the level of a receptor protein of the invention or a partial peptide thereof in a cell membrane can be accomplished by:

(i) administering a test compound at a certain time point before (30 minutes to 24 hours, preferably 30 minutes to 12 hours, more preferably 1 hour to 6 hours before) or at a certain time point after (30 minutes to 3 days, preferably 1 hour to 2 days, more preferably 1 hour to 24 hours after) subjecting a normal or disease-bearing non-human mammalian model animal to a medication or a mechanical stress, or simultaneously with a medication or a mechanical stress, followed by quantifying the level of the receptor protein of the invention or the partial peptide thereof contained in a cell at a certain time point after (30 minutes to 3 days, preferably 1 hour to 2 days, more preferably 1 hour to 24 hours after) the administration;

(ii) adding a test compound to a culture medium in which a transformant is incubated by a standard method followed by quantifying the level of the receptor protein of the invention or the partial peptide thereof contained in a cell membrane at a certain time point after (1 day to 7 days, preferably 1 day to 3 days, more preferably 2 days to 3 days after) initiation of the incubation.

Typically, the level of a receptor protein of the invention or a partial peptide thereof contained in a cell membrane fraction is determined as follows.

(iii) A normal or a disease-bearing non-human mammalian model animal (for example, mouse, rat, rabbit, sheep, swine, cattle, cat, dog, monkey and the like, more specifically, dementia rat, obesity mouse, arteriosclerosis rabbit, bile cancer mouse and the like) is treated with an agent (for example, anti-dementia agent, hypotensive agent, anti-cancer agent, anti-obesity agent and the like) or subjected to a mechanical stress (for example, flooding stress, electric shock, dark/light cycle, low temperature and the like), and after a certain time period, blood, a certain organ (for example, brain, liver, kidney and the like) or a tissue isolated from an organ or a cell is collected. The organ, tissue or cell thus obtained is cut into sections by a standard method, and subjected to an immunostaining using an antibody of the invention. By quantifying the staining of the receptor protein in the surface layer of the cell to identify the protein on the cell membrane, a verification of the level of the receptor protein of the invention or the partial peptide thereof in the cell membrane is possible quantitatively and qualitatively.

(iv) A verification is possible also by subjecting a transformant expressing a receptor protein or a salt thereof to the similar procedure.

A compound or a salt thereof obtained by a screening method of the invention is a compound having an ability of altering the level of a receptor protein of the invention or a partial peptide thereof in a cell membrane, and typically it is (a) a compound which enhances a G-protein coupled receptor protein-mediated cell stimulating activity (for example, an activity which promotes or suppresses arachidonic acid release, acetylcholin release, intracellular $Ca^{2+}$ release, intracellular cAMP production, intracellular cGMP production, inositol phosphate production, cell membrane potential variation, intracellular protein phosphorylation, c-fos activation, pH reduction and the like) by increasing the level of a receptor protein of the invention or a partial peptide thereof in a cell membrane, and (b) a compound which suppresses such cell stimulating activity by reducing the level of a receptor protein of the invention or a partial peptide thereof in a cell membrane.

Such compound may for example be a peptide, a protein, a non-peptide compound, a synthetic compound, a fermented compound and the like, which may be novel compounds or known compounds.

A compound enhancing such cell stimulating activity is useful as a safe and pharmaceutical low in toxicity for enhancing the physiological activity of a receptor protein of the invention.

A compound suppressing such cell stimulating activity is useful as a safe and pharmaceutical low in toxicity for reducing the physiological activity of a receptor protein of the invention.

When a compound or a salt thereof obtained by a screening method of the invention is used in a pharmaceutical composition, a standard procedure may be followed. For example, similarly to a pharmaceutical containing a receptor protein of the invention, a dosage form such as tablet, capsule, elixir, microcapsule, aseptic solution or suspension can be formulated.

Since a formulation thus obtained is safe and low in toxicity, it can be administered to a human or a mammalian animal (for example, rat, rabbit, sheep, swine, cattle, cat, dog, monkey and the like).

While such compound or a salt thereof may be given at a dose which varies depending on the patient to be treated, the target organ, the condition of the patient and the administration route, it can be given orally to an adult (60 kg) usually at a daily dose of about 0.1 mg to 100 mg, preferably about 1.0 to 50 mg, more preferably about 1.0 to 20 mg. When it is given parenterally, the single dose may vary depending on the patient to be treated, the target organ, the condition of the patient and the administration route, and may for example be given as a formulation for injection to an adult (60 kg) usually at a daily dose of about 0.01 mg to 30 mg, preferably about 0.1 to 20 mg, more preferably about 0.1 to 10 mg, which is given advantageously by an intravenous injection. Also in other animals, a dose calculated for a 60 kg body weight may be administered.

(11) Preventing and/or treating agent against various diseases containing compound capable of altering amount of receptor protein of the invention or partial peptide, thereof in cell membrane

As described above, a receptor protein of the invention is considered to play some important in vivo role for example in central nervous function. Accordingly, a compound altering the amount of a receptor protein of the invention or a partial peptide thereof in a cell membrane can be used as a preventing and/or treating agent against a disease related to the dysfunction of the receptor protein of the invention.

When such compound is used as a preventing and/or treating agent against a disease related to the dysfunction of a receptor protein of the invention, a standard formulation procedure may be followed.

For example, such compound may be given orally as an optionally sugar-coated tablet, capsule, elixir, microcapsule and the like, or parenterally as a formulation for injection such as an aseptic solution or suspension in water or pharmacologically acceptable liquid. For example, such formulation can be produced by mixing the compound with a known physiologically acceptable carrier, flavor, excipient, vehicle, preservative, stabilizer, binder and the like in a unit dosage form which is acceptable generally in pharmaceutical practice. The amount of the active ingredient in such formulation should be adjusted to achieve a suitable dose within a specified range.

An additive which may be incorporated into a tablet or a capsule may for example be a binder such as gelatin, corn starch, tragacanth, gum arabic and the like, an excipient such as crystalline cellulose, an expander such as corn starch, gelatin, alginic acid and the like, a lubricant such as magnesium stearate, a sweetener such as sucrose, lactose or saccharin, a flavor such as peppermint, oil of Geultheria ovatifolia spp., cherry and the like. When a unit dosage form is a capsule, a liquid carrier such as a fat may further be incorporated in addition to the materials described above. An aseptic formulation for injection can be prepared in accordance with ordinary pharmaceutical practice such as a dissolution or a suspension of an active ingredient, a naturally-occurring vegetable oil such as sesame oil and palm oil in avehicle such as water for injection. An aqueous liquid for injection may for example be physiological saline, an isotonic solution containing glucose or other auxiliary agents (for example, D-sorbitol, D-mannitol, sodium chloride) and the like, which may be used in combination with a suitable solubilizer such as an alcohol (for example, ethanol), a polyalcohol (for example, propylene glycol, polyethylene glycol), a non-ionic surfactant (for example, polysorbate 80™, HCO-50). An oily liquid may for example be sesame oil and soybean oil, which may be used in combination with an solubilizer such as benzyl benzoate, benzyl alcohol and the like.

In addition, a preventing and/or treating agent described above may be supplemented also with a buffer agent (for example, phosphate buffer, sodium acetate buffer), a pain-ameliorating agent (for example, benzalkonium chloride, procaine hydrochloride), a stabilizer (for example, human serum albumin, polyethylene glycol), a preservative (for example, benzyl alcohol, phenol), an antioxidant. A formulation for injection thus prepared is then usually filled in a suitable ampoule.

Since a formulation thus obtained is safe and low in toxicity, it can be administered to a human or a mammalian animal (for example, rat, rabbit, sheep, swine, cattle, cat, dog, monkey and the like).

While such compound or a salt thereof may be given at a dose which varies depending on the patient to be treated, the target organ, the condition of the patient and the administration route, it can be given orally to an adult (60 kg) usually at a daily dose of about 0.1 mg to 100 mg, preferably about 1.0 to 50 mg, more preferably about 1.0 to 20 mg. When it is given parenterally, the single dose may vary depending on the patient to be treated, the target organ, the condition of the patient and the administration route, and may for example be given as a formulation for injection to an adult (60 kg) usually at a daily dose of about 0.01 mg to 30 mg, preferably about 0.1 to 20 mg, more preferably about 0.1 to 10 mg, which is given advantageously by an intravenous injection. Also in other animals, a dose calculated for a 60 kg body weight may be administered.

(12) Neutralization of receptor protein of the invention or partial peptide thereof or salt thereof by antibody

The neutralizing activity of an antibody against a receptor protein of the invention or a partial peptide thereof or a salt thereof means an activity by which a signal transmission function involving such receptor protein and the like is inactivated. Accordingly, when such antibody has a neutralizing activity, a signal transmission involving such receptor protein, such as the receptor protein-mediated cell stimulating activity (for example, an activity which promotes or suppresses arachidonic acid release, acetylcholin release, intracellular $Ca^{2+}$ release, intracellular cAMP production, intracellular cGMP production, inositol phosphate production, cell membrane potential variation, intracellular protein phosphorylation, c-fos activation, pH reduction and the like) can be inactivated. As a result, an application to the prevention and/or the treatment of a disease caused for example by an overexpression of such receptor protein is possible.

(13) Creation of non-human animal having DNA encoding G-protein coupled receptor protein of the invention

A transgenic non-human animal expressing a receptor protein and the like of the invention can be created using a DNA of the invention. Such non-human animal may for example be a mammalian animal (for example, rat, mouse, rabbit, sheep, swine, cattle, cat, dog, monkey and the like) and the like (hereinafter abbreviated as an animal), withmouse and rabbit being preferred particularly.

When a DNA of the invention is transferred to a target animal, a use as a gene construct bound downstream of a promoter capable of expressing such DNA in an animal cell is usually advantageous. When a DNA of the invention which is derived for example from a rabbit is to be transferred, a gene construct bound downstream of any promoter capable of expressing a DNA of the invention derived from an animal having a high homology thereto in an animal cell is microinjected for example to a fertilized ovum of a rabbit to create a DNA transfer animal producing a receptor protein and the like of the invention at a high level. While such promoter may for example be a ubiquitous expression promoter such as virus-derived promoter and metallothioneine, an NGF gene promoter expressing specifically in a brain or an enolase gene promoter are employed preferably.

The transfer of a DNA of the invention at the stage of a fertilized ovum is preserved to be present throughout the entire embryonic and somatic cells in a target animal. The presence of a receptor protein and the like of the invention in an embryonic cell of a created animal after the DNA transfer means that the entire descendants of the created animal will have the receptor protein and the like of the invention in their embryonic and somatic cells. Thus, a descendant of this species taking over this gene will have the receptor protein and the like of the invention in all of its embryonic and somatic cells.

A DNA transfer animal of the invention, once ensuring a stable preservation of the gene even after a mating, can be subjected as a relevant DNA-possessing animal to a hereditary breeding under an ordinary breeding condition. Furthermore, by mating the animals of the both sexes each having an intended DNA, a homozygote animal having the transduced genes in the both of their homologous chromosomes can be obtained, and then by mating the animals of the both sexes thus obtained a hereditary breeding to allow all descendants to have the relevant DNA is possible.

Since an animal to which a DNA of the invention has been transferred allows a receptor protein and the like of the invention to be highly expressed, it is useful as an animal for screening for an agonist or an antagonist for the receptor protein and the like of the invention.

A DNA transfer animal of the invention can be used also as a cell source for a tissue culture. For example, by analyzing a DNA or an RNA in a tissue of a DNA transfer mouse of the invention directly or analyzing a tissue having a receptor protein of the invention expressed by the gene, the receptor protein and the like of the invention can be investigated. A cell of a tissue having a receptor protein of the invention is incubated by a standard tissue culture technology and used to investigate the function of a cell from a tissue which is generally difficult to be incubated, such as a brain and a peripheral tissue. Also by using such cell, a pharmaceutical capable of promoting the function of each tissue can be selected. Furthermore, a receptor protein and the like of the invention can be isolated and purified from a highly expressing cell.

In the specification and the drawings, a base or an amino acid may be designated as a code based on IUPAC-IUB, Commission on Biological Nomenclature or as a customary abbreviation in the art as exemplified below. When an amino acid is present as an optical isomer, it is in L form unless otherwise specified.

DNA :    Deoxyribonucleic acid
cDNA :   Complementary deoxyribonucleic acid
A :        Adenine
T :        Thymine
G :       Guanine
C :       Cytosine
RNA :    Ribonucleic acid
mRNA :   Messenger ribonucleic acid
dATP :    Deoxyadenosine triphosphate
dTTP :    Deoxythymidine triphosphate

dGTP : Deoxyguanosine triphosphate
dCTP : Deoxycytidine triphosphate
ATP : Adenosine triphosphate
EDTA : Ethylenediamine tetraacetic acid
SDS : Sodium dodecylsulfate
Gly : Glycine
Ala : Alanine
Val : Valine
Leu : Leucine
Ile : Isoleucine
Ser : Serine
Thr : Threonine
Cys : Cysteine
Met : Methionine
Glu : Glutamic acid
Asp : Aspartic acid
Lys : Lysine
Arg : Arginine
His : Histidine
Phe : Phenylalanine
Tyr : : Tyrosine
Trp : Tryptophan
Pro : Proline
Asn : Asparagine
Gln : Glutamine
pGlu : Pyroglutaminic acid
Me : Methyl group
Et : Ethyl group
Bu : Butyl group
Ph : Phenyl group
TC : Thiazolidine-4 (R) -carboxamide group

Substituents, protective groups and reagents employed frequently in the specification are represented by the following codes.

Tos p-Toluenesulfonyl
CHO : Formyl
Bzl : Benzyl
$Cl_2Bzl$ : 2,6-Dichlorobenzyl
Bom : Benzyloxymethyl
Z : Benzyloxycarbonyl
Cl-Z : 2-Chlorobenzyloxycarbonyl
Br-Z : 2-Bromobenzyloxycarbonyl
Boc : t-Butoxycarbonyl
DNP : Dinitrophenyl
Trt : Trityl
Bum : t-Butoxymethyl
Fmoc : N-9-Fluorenylmethoxycarbonyl
HOBt : 1-Hydroxybenztriazole
HOOBt : 3,4-Dihydro-3-hydroxy-4-oxo-1,2,3-benzotriazine
HONB : 1-Hydroxy-5-norbornene-2,3-dicarboxyimide
DCC : N,N'-Dicyclohexylcarbodiimide

The SEQ. ID. No. in the sequence listing in this specification represent the following sequences.

[SEQ ID NO.1]

SEQ ID NO.1 represents an amino acid sequence of a human hippocampus-derived novel G-protein coupled receptor protein H7TMB56 of the invention.

[SEQ ID NO.2]

SEQ ID NO.2 represents a base sequence of a cDNA encoding a human hippocampus-derived novel G-protein coupled receptor protein H7TMB56 of the invention having the amino acid sequence represented by SEQ ID NO.1.

[SEQ ID NO.3]

SEQ ID NO.3 represents a base sequence of a cDNA encoding a human hippocampus-derived novel G-protein coupled receptor protein H7TMB56 of the invention having the amino acid sequence represented by SEQ ID NO.1.

[SEQ ID NO.4]

SEQ ID NO.4 represents a base sequence of Primer 1 employed for cloning a cDNA encoding a human hippocampus-derived novel G-protein coupled receptor protein H7TMB56 of the invention.

[SEQ ID NO.5]

SEQ ID NO.5 represents a base sequence of Primer 2 employed for cloning a cDNA encoding a human hippocampus-derived novel G-protein coupled receptor protein H7TMB56 of the invention.

[SEQ ID NO.6]

SEQ ID NO.6 represents an amino acid sequence of a human hippocampus-derived novel G-protein coupled receptor protein H7TMB56 of the invention.

[SEQ ID NO.7]

SEQ ID NO.7 represents a base sequence of a cDNA encoding a G-protein coupled receptor protein having the amino acid sequence represented by SEQ ID NO.6.

A transformant Escherichia coli DH5α/pCRII-H7TMB56 obtained in Example 1 described below has been deposited to National Institute of Bioscience and Human Technology of Agency of Industrial Science and Technology of the Ministry of International Trade and Industry (NIBH) on September 4, 1998 under the deposition number FERM BP-6483 and also to Institute for Fermentation (IFO) on June 19, 1998 under the deposition No.IFO 16186.

The present invention is further described in detail in the following Examples, which are not intended to restrict the invention. A gene engineering procedure employing E.coli was in accordance with the methods described in Molecular Cloning.

Example 1 Cloning and base sequencing of cDNA encoding human hippocampus-derived G-protein coupled receptor protein (1)

A PCR was performed using a human hippocampus cDNA (Marathon-Ready™ cDNA, CLONTECH) as a template together with two primers, namely, Primer 1 (SEQ ID NO.4) and Primer 2 (SEQ ID NO.5). The reaction mixture in this PCR contained a 1/10 volume of the cDNA described above as a template, a 1/50 volume of Advantage cDNA polymerase Mix (CLONTECH), each 0.2 µM of Primer 1 (SEQ ID NO.4) and Primer 2 (SEQ ID NO.5), 200 µM of dNTPs together with a buffer attached to the enzyme, in the total volume of 25 µL. The PCR involved [1] 1 minute at 95°C, [2] three cycles of 20 seconds at 94° C followed by 2 minutes at 72°C, [3] three cycles of 20 seconds at 94°C followed by 2 minutes at 68°C, [4] 38 cycles of 20 seconds at 94°C followed by 20 seconds at 63.5°C followed by 2 minutes and 20 seconds at 68°C and [5] a final chain elongation for 7 minutes at 68°C. After this PCR, the reaction product was subcloned to a plasmid vector pCRII (INVITROGEN) according to the instruction attached to a TA cloning kit (INVITROGEN). The vector was then transduced into E.coli DH5α, and a clone having a cDNA was screened for in an LB agar medium containing ampicillin, and then each clone was sequenced to obtain a cDNA sequence (SEQ ID Nos.2 and 3) encoding a novel G-protein coupled receptor protein. A novel G-protein coupled receptor protein containing the amino acid sequence (SEQ ID NO.1) derived from this cDNA was designated as H7TMB56.

A plasmid pCRII-H7TMB56 subcloned with a cDNA (SEQ ID NO.2) encoding a human hippocampus-derived G-protein coupled receptor protein H7TMB56 of the invention was transduced into E. coli DH5α by a method known per se to obtain a transformant E.coli DH5α/ pCRII-H7TMB56.

Example 2 Cloning and base sequencing of cDNA encoding human hippocampus-derived G-protein coupled receptor protein (2)

A PCR was performed using a human hippocampus cDNA (Marathon-Ready™ cDNA, CLONTECH) as a template together with two primers, namely, Primer 1 (SEQ ID NO.4) and Primer 2 (SEQ ID NO.5). The reaction mixture in this PCR contained a 1/10 volume of the cDNA described above as a template, a 1/50 volume of Advantage cDNA polymerase Mix (CLONTECH), each 0.2 µM of Primer 1 (SEQ ID NO.4) and Primer 2 (SEQ ID NO.5), 200 µM of dNTPs together with a buffer attached to the enzyme, in the total volume of 25 µL. The PCR involved [1] 1 minute at 95°C, [2] three cycles of 20 seconds at 94° C follovled by 2 minutes at 72°C, [3] three cycles of 20 seconds at 94°C followed by 2 minutes at 68°C, [4] 38 cycles of 20 seconds at 94°C followed by 20 seconds at 63.5°C followed by 2 minutes and 20 seconds at 68°C and [5] a final chain elongation for 7 minutes at 68°C. After this PCR, the reaction product was subcloned to a plasmid vector pCRII (INVITROGEN) according to the instruction attached to a TA cloning kit (INVITROGEN). The vector was then transduced into E.coli DH5α, and a clone having a cDNA was screened for in an LB agar medium containing ampicillin, and then each clone was sequenced to obtain a cDNA sequence (SEQ ID No.7) encoding a novel G-protein coupled receptor protein. A novel G-protein coupled receptor protein-containing the amino acid sequence (SEQ ID NO.6) derived from this cDNA was designated as H7TMB56.

Example 3 Cloning and base sequencing of cDNA encoding human genome-derived G-protein coupled receptor protein (1)

A PCR was performed using a human genome DNA (about 1 ng/µl) as a template together with two primers, namely, Primer 1 (SEQ ID NO.4) and Primer 2 (SEQ ID NO.5). The reaction mixture in this PCR contained a 1/25 volume of the genome DNA described above as a template, a 1/50 volume of Advantage cDNA polymerase Mix (CLONTECH), each 0.2 µM of Primer 1 (SEQ ID NO.4) and Primer 2 (SEQ ID NO.5), 200 µ M of dNTPs together with a buffer attached to the enzyme, in the total volume of 25 µL. The PCR involved [1] 1 minute at 94°C, [2] four cycles of 20 seconds at 94°C followed by 2 minutes at 72°C, [3] four cycles of 20 seconds at 94°C followed by 2 minutes at 70°C, [4] 27 cycles of 20 seconds at 94°C followed by 2 minutes at 68°C and [5] a final chain elongation for 7 minutes at 68°C. After this PCR, the reaction product was subcloned to a plasmid vector pCRII (INVITROGEN) according to the instruction attached to a TA cloning kit (INVITROGEN). The vector was then transduced into E.coli DH5α, and a clone having a genome fragment was screened for in an LB agar medium containing ampicillin, and then each clone was sequenced to ensure that the base sequence was identical to the DNA sequences (SEQ ID NOs. 2, 3 and 7) each encoding a novel G-protein coupled receptor protein obtained in Examples 1 and 2.

Example 4 Preparation of H7TMB56- and H7TMB56C-expressing CHO cell

Similarly to Example 1, a plasmid pCRII-H7TMB56 subcloned with a cDNA (SEQ ID NO.7) encoding H7TMB56C was transduced into E.coli DH5α by a method known per se to obtain a transformant E.coli DH5α/ pCRII-H7TMB56C.

A transformant E.coli DH5α/ pCRII-H7TMB56 obtained in Example 1 or a transformant E.coli DH5α/ pCRII-H7TMB56C was incubated and then PLASMIDMIDKIT (QUIAGENE) was employed to prepare a plasmid DNA of pCRII-H7TMB56 or pCRII-H7TMB56C. From each of these plasmids, a cDNA encoding a G-protein coupled receptor protein H7TMB56 or H7TMB56C of the invention was cloned to a protein-expressing plasmid vector pcDNA3.1/V5/His to construct a protein-expressing plasmid pcDNA3.1-H7TMB56 or -H7TMB56C. The plasmid thus obtained was subjected to PLASMIDMIDKIT (QUIAGENE) to produce a large amount of the plasmid DNA, which was then transduced into a CHO dhfr- cell using CELLFECT TRANSFECTION KIT (AMERSHAM PHARMACIA BIOTECH) according to the protocol attached thereto. Thus, 10 mg of the DNA was co-suspended with calcium phosphate and added to a 10 cm petri dish inoculated 24 hours ago with $5 \times 10^5$ or $1 \times 10^6$ CHO dhfr- cells, and incubated for one day in a MEMα medium supplemented with 10 % fetal calf serum and then subcultured and then incubated in a selection medium which was a MEMα medium supplemented with 0.4mg/ml G418 (GIBCO BRL) and 10 % dialyzed fetal calf serum. By selecting a colony of a transformant cell (CHO/H7TMB56 or CHO/H7TMB56C) growing in the selection medium, a H7TMB56-or H7TMB56C-expressing CHO cell was obtained.

After extracting a total RNA from the selected H7TMB56-expressing CHO cell by a standard method, the amount of the mRNA of H7TMB56 was determined and the number of the copies was calculated by a TaqMan method. The results are shown below.

Table 1

| Clone No | Expression level (copy/ng total RNA) | |
|---|---|---|
| | 1st measurement | 2nd measurement |
| 7 | 83046 | 89931 |
| | 104954 | 71409 |
| 9 | 32632 | 30669 |
| | 61153 | 28487 |
| 15 | 5146 | 7970 |
| | 7172 | 4882 |

Industrial Applicability

A G-protein coupled receptor protein of the invention or a salt thereof, a partial peptide and a salt thereof, and a polynucleotide encoding such G-protein coupled receptor protein or a partial peptide thereof (for example, DNA, RNA or its derivative) is useful in [1] determining a ligand (agonist), [2] obtaining an antibody and an antiserum, [3] constructing a recombinant receptor protein expression system, [4] developing a receptor binding assay system and screening for a pharmaceutical candidate compound using this expression system, [5] designing a drug based on the comparison with a structurally-related ligand/receptor, [6] a reagent for preparing a probe or a PCR primer in a gene diagnosis, [7] creating a transgenic animal, or [8] a pharmaceutical such as a genetically preventing or treating agent.

**Claims**

1.  A G-protein coupled receptor protein comprising an amino acid sequence identical or substantially identical to the amino acid sequence represented by SEQ ID NO.1 or a salt thereof.

2.  A G-protein coupled receptor protein according to Claim 1 wherein an amino acid sequence substantially identical to the amino acid sequence represented by SEQ ID NO.1 is the amino acid sequence represented by SEQ ID NO. 6 or a salt thereof.

3.  Apartial peptide of a G-protein coupled receptor protein according to Claim 1 or a salt thereof.

4.  A polynucleotide comprising a polynucleotide having a base sequence encoding a G-protein coupled receptor protein according to Claim 1.

5.  A polynucleotide according to Claim 4 which is a DNA.

6.  A polynucleotide according to Claim 4 having the base sequence represented by SEQ ID NO.2 or SEQ ID NO.3.

7.  A polynucleotide according to Claim 4 comprising a polynucleotide having a base sequence encoding a G-protein coupled receptor protein comprising the amino acid sequence represented by SEQ ID NO.6.

8.  A polynucleotide according to Claim 7 which is a DNA.

9.  A polynucleotide according to Claim 7 having the base sequence represented by SEQ ID NO.7.

10. A recombinant vector comprising a polynucleotide according to Claim 4.

11. A transformant transformed with a recombinant vector according to Claim 10.

12. A method for producing a G-protein coupled receptor protein according to Claim 1 or a salt thereof comprising incubating a transformant according to Claim 11 to produce a G-protein coupled receptor protein according to Claim 1.

13. An antibody directed to a G-protein coupled receptor protein according to Claim 1 or a partial peptide according to Claim 3 or a salt thereof.

14. An antibody according to Claim 13 which is a neutralizing antibody which inactivates the signal transmission of a G-protein coupled receptor protein according to Claim 1.

15. A diagnostic agent comprising an antibody according to Claim 13.

16. A ligand for a G-protein coupled receptor protein according to Claim 1 or a salt thereof obtained by using a G-protein coupled receptor protein according to Claim 1 or a partial peptide according to Claim 3 or a salt thereof.

17. A pharmaceutical comprising a ligand for a G-protein coupled receptor protein according to Claim 16.

18. A method for determining a ligand for a G-protein coupled receptor protein according to Claim 1 or a salt thereof comprising using a G-protein coupled receptor protein according to Claim 1 or a partial peptide according to Claim 3 or a salt thereof.

19. A method for screening for a compound or a salt thereof which is capable of altering the binding affinity between a ligand and a G-protein coupled receptor protein according to Claim 1 or a salt thereof comprising using a G-protein coupled receptor protein according to Claim 1 or a partial peptide according to Claim 3 or a salt thereof.

20. A kit for screening for a compound or a salt thereof capable of altering the binding affinity between a ligand and a G-protein coupled receptor protein according to Claim 1 or a salt thereof comprising using a G-protein coupled receptor protein according to Claim 1 or a partial peptide according to Claim 3 or a salt thereof.

21. A compound or a salt thereof capable of altering the binding affinity between a ligand and a G-protein coupled receptor protein according to Claim 1 or a salt thereof which can be obtained by using a screening method according to Claim 19 or a screening kit according to Claim 20.

22. A pharmaceutical comprising a compound or a salt thereof capable of altering the binding affinity between a ligand and a G-protein coupled receptor protein according to Claim 1 or a salt thereof which can be obtained by usinga screening method according to Claim 19 or a screening kit according to Claim 20.

23. A polynucleotide capable of being hybridized with a polynucleotide according to Claim 4 under a high stringent condition.

24. A polynucleotide comprising a base sequence or a part thereof which is complementary with a polynucleotide according to Claim 4.

25. A method for quantifying an mRNA of a G-protein coupled receptor protein according to Claim 1 comprising using a polynucloeotide according to Claim 4 or a part thereof.

26. A method for quantifying a G-protein coupled receptor protein according to Claim 1 comprising using an antibody according to Claim 13.

27. A method for diagnosing a disease related to a function of a G-protein coupled receptor protein according to Claim 1 comprising using a quantification method according to Claim 25 or Claim 26.

28. A method for screening for a compound or a salt thereof capable of altering the expression level of a G-protein coupled receptor protein according to Claim 1 comprising using a quantification method according to Claim 25.

29. A method for screening for a compound or a salt thereof capable of altering the amount of a G-protein coupled receptor protein according to Claim 1 in a cell membrane comprising using a quantification method according to Claim 26.

30. A compound or a salt thereof capable of altering the expression level of a G-protein coupled receptor protein according to Claim 1 which can be obtainedby using a screening method according to Claim 28.

**31.** A compound or a salt thereof capable of altering the amount of a G-protein coupled receptor protein according to Claim 1 in a cell membrane which can be obtained by using a screening method according to Claim 29:

Figure 1

Parameter        : Kyte & Doolittle
Range to Average : 15

Figure 2

```
 37  ISLVGLTGNAVVLWLLGYRMRRNAVSIYILNLAAAD..FLFLSFQIIRSP 84
     || :|:: |:::||:| :||||| ::|| :|: ||  :||  | |:
 41  ISPLGFVENGILLWFLCFRMRRNPFTVYITHLSIADISLLFCIF.ILSID 89


 85  LRL...INISHLIRKILVSV.MTFPYFTGLSMLSAISTERCLSVLWPIWY 130
     |    :: :|    : :|| : | | ||| :|:|||:|||||||:||||
 90  YALDYELSSGHYYTIVTLSVTFLFGYNTGLYLLTAISVERCLSVLYPIWY 139


131  RCRRPTHLSAVVCVLLWGLSLLFSMLEWRFCDFLFSGADSSWCETSD... 177
     ||:|| | || ||:|||:|| | :.:|: :| : || :|  : ||
140  RCHRPKHQSAFVCALLWALSCLVTTMEYVMC..IDSGEESH..SQSDCRA 185


178  ...FIPVAWLIFLCVVLCVSSLVLLVRILCGSRKMPLTRLYVTILLTVLV 224
        || : :: : :: ||| :|:|:| ::     ::||::|::|:::
186  VIIFIAILSFLVFTPLMLVSSTILVVKIRKNTWASHSSKLYIVIMVTIII 235


225  FLLCGLPFGILGALIY.......RMHLNLEVLYCHVYLVCMSLSSLNSSA 267
     ||: ::|: :| | |       ::| |:::|:      |::||||
236  FLIFAMPMRVLYLLYYEYWSTFGNLH.NISLLF..........STINSSA 274


268  NPIIYFFVGSFRQRQNRQNLKLVLQRALQDKPEVDKGEGQLPEESLE 314
     ||:|||||||| :::: |::||:|| ||::|: :  : ||:    |:|
275  NPFIYFFVGSSKKKRFRESLKVVLTRAFKDEMQPRRQEGNGNTVSIE 321
```

Figure 3

```
  1   ATGGATCCAACCGTCCCAGTCTTGGGTACAAAACTGACACCAATCAACGGACGTGAGGAG   60
      MetAspProThrValProValLeuGlyThrLysLeuThrProIleAsnGlyArgGluGlu

 61   ACTCCTTGCTACAAGCAGACCCTGAGCTTCACGGTGCTGACGTGCATCATTTCCCTTGTC  120
      ThrProCysTyrLysGlnThrLeuSerPheThrValLeuThrCysIleIleSerLeuVal

121   GGACTGACAGGAAACGCGGTTGTGCTCTGGCTCCTGGGCTGCCGCATGCGCAGGAACGCT  180
      GlyLeuThrGlyAsnAlaValValLeuTrpLeuLeuGlyCysArgMetArgArgAsnAla

181   GTCTCCATCTACATCCTCAACCTGGCCGCAGCAGACTTCCTCTTCCTCAGCTTCCAGATT  240
      ValSerIleTyrIleLeuAsnLeuAlaAlaAlaAspPheLeuPheLeuSerPheGlnIle

241   ATACGTTCGCCATTACGCCTCATCAATATCAGCCATCTCATCCGCAAAATCCTCGTTTCT  300
      IleArgSerProLeuArgLeuIleAsnIleSerHisLeuIleArgLysIleLeuValSer

301   GTGATGACCTTTCCCTACTTTACAGGCCTGAGTATGCTGAGCGCCATCAGCACCGAGCGC  360
      ValMetThrPheProTyrPheThrGlyLeuSerMetLeuSerAlaIleSerThrGluArg

361   TGCCTGTCTGTTCTGTGGCCCATCTGGTACCGCTGCCGCCGCCCCACACACCTGTCAGCG  420
      CysLeuSerValLeuTrpProIleTrpTyrArgCysArgArgProThrHisLeuSerAla

421   GTCGTGTGTGTGTCCTGCTCTGGGGCCTGTCCCTGCTGTTTAGTATGCTGGAGTGGAGGTTC  480
      ValValCysValLeuLeuTrpGlyLeuSerLeuLeuPheSerMetLeuGluTrpArgPhe
```

# Figure 4

481 TGTGACTTCCTGTTTAGTGGTGCTGATTCTAGTTGGTGTGAAACGTCAGATTTCATCCCA 540
CysAspPheLeuPheSerGlyAlaAspSerSerTrpCysGluThrSerAspPheIlePro

541 GTCGCGTGGCTGATTTTTTTATGTGTGTGGTTCTCTGTGTTTCCAGCCTGGTCCTGCTGGTC 600
ValAlaTrpLeuIlePheLeuCysValValLeuCysValSerSerLeuValLeuLeuVal

601 AGGATCCTCTGTGGATCCCGGAAGATGCCGCTGACCAGGCTGTACGTGACCATCCTGCTC 660
ArgIleLeuCysGlySerArgLysMetProLeuThrArgLeuTyrValThrIleLeuLeu

661 ACAGTGCTGGTCTTCCTCCTCTGCGGCCTGCCCTTCGGCATTCTGGGGGGCCCTAATTTAC 720
ThrValLeuValPheLeuLeuCysGlyLeuProPheGlyIleLeuGlyAlaLeuIleTyr

721 AGGATGCACCTGAATTTGGAAGTCTTATATTGTCATGTTTATCTGGTTTGCATGTCCCTG 780
ArgMetHisLeuAsnLeuGluValLeuTyrCysHisValTyrLeuValCysMetSerLeu

781 TCCTCTCTAAACAGTAGTGCCAACCCCATCATTTACTTCTTCGTGGGCTCCTTTAGGCAG 840
SerSerLeuAsnSerSerAlaAsnProIleIleTyrPhePheValGlySerPheArgGln

841 CGTCAAAATAGGCAGAACCTGAAGCTGGTTCTCCAGAGGGCTCTGCAGGACAAGCCTGAG 900
ArgGlnAsnArgGlnAsnLeuLysLeuValLeuGlnArgAlaLeuGlnAspLysProGlu

901 GTGGATAAAGGTGAAGGGCAGCTTCCTGAGGAAAGCCTGGAGCTGTCGGGAAGCAGATTG 960
ValAspLysGlyGluGlyGlnLeuProGluGluSerLeuGluLeuSerGlySerArgLeu

961 GGGCCATGA 969
GlyPro***

## INTERNATIONAL SEARCH REPORT

| | International application No. |
|---|---|
| | PCT/JP99/05365 |

A. CLASSIFICATION OF SUBJECT MATTER
    Int.Cl$^7$   C07K14/705, 16/28, C12N1/21, 15/12, C12P21/02,
              G01N33/68, A61K38/00

According to International Patent Classification (IPC) or to both national classification and IPC

B. FIELDS SEARCHED

Minimum documentation searched (classification system followed by classification symbols)
    Int.Cl$^7$   C07K14/00-14/825, C12N15/11-15/62

Documentation searched other than minimum documentation to the extent that such documents are included in the fields searched

Electronic data base consulted during the international search (name of data base and, where practicable, search terms used)
    GenBank/EMBL/DDBJ/GeneSeq, SwissProt/PIR/GeneSeq,
    WPI(DIALOG), BIOSIS(DIALOG)

C. DOCUMENTS CONSIDERED TO BE RELEVANT

| Category* | Citation of document, with indication, where appropriate, of the relevant passages | Relevant to claim No. |
|---|---|---|
| X<br>A | Cell, Vol. 45, No. 5, issued 06 June, 1986, Dallan Young et al., "Isolation and Characterization of a New Cellular Oncogene Encoding a Protein with Multiple Potential Trans-membrane Domains", pages 711-719 | 24<br>1-23,25,26,<br>28-31 |
| X<br>A | Molecular Endocrinology, Vol. 6, No. 12, issued December, 1992, Yuichiro Yamada et al., 'Somatostatin Receptors, an Expanding Gene Family: Cloning and Functional Characterization of Human SSTR3, a Protein Coupled to Adenylyl Cyclase", pages 2136-2142 | 24<br>1-23,25,26,<br>28-31 |
| X<br>A | FEBS Letters, Vol. 321, Nos. 2,3, issued 26 April, 1993, Jacquie D. Corness et al., "A human somatostatin receptor (SSTR3), located on chromosome 22, displays preferential affinity for somatostatin-14 like peptides", pages 279-284 | 24<br>1-23,25,26,<br>28-31 |

☒ Further documents are listed in the continuation of Box C.　　☐ See patent family annex.

| | |
|---|---|
| *    Special categories of cited documents:<br>"A"   document defining the general state of the art which is not considered to be of particular relevance<br>"E"   earlier document but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search<br>    11 January, 2000 (11.01.00) | Date of mailing of the international search report<br>    25 January, 2000 (25.01.00) |
|---|---|
| Name and mailing address of the ISA/<br>    Japanese Patent Office | Authorized officer |
| Facsimile No. | Telephone No. |

Form PCT/ISA/210 (second sheet) (July 1992)

## INTERNATIONAL SEARCH REPORT

| International application No. |
|---|
| PCT/JP99/05365 |

C (Continuation). DOCUMENTS CONSIDERED TO BE RELEVANT

| Category* | Citation of document, with indication, where appropriate, of the relevant passages | Relevant to claim No. |
|---|---|---|
| X<br>A | Proceedings of the National Academy of Sciences, USA, Vol. 89, No. 1, issued 01 January, 1992, Yuichiro Yamada et al., "Cloning and functional characterization of a family of human and mouse somatostatin receptors expressed in brain, gastrointestinal tract, and kidney", pages 251-255 | 24<br>1-23,25,26, 28-31 |
| A | Molecular Endocrinology, Vol. 5, No. 10, issued October, 1991, C. Monnot et al., "Cloning and Functional Characterization of a Novel mas-Related Gene, Modulating Intracellular Angiotensin II Actions", pages 1477-1487 | 1-26, 28-31 |
| A | Proceedings of the National Academy of Sciences, USA, Vol. 85, No. 14, issued July 1988, Dallan Young et al., "Characterization of the rat mas oncogene and its high-level expression in the hippocampus and cerebral cortex of rat brain", pages 5339-5342 | 1-26, 28-31 |
| A | WO, 96/16087, A1 (HUMAN GENOME SCIENCES, INC.), 30 May, 1996 (30.05.96)<br>& AU, 9515501, A  & ZA, 9409667, A<br>& EP, 871667, A1  & JP, 10-509870, A | 1-26, 28-31 |

Form PCT/ISA/210 (continuation of second sheet) (July 1992)

**INTERNATIONAL SEARCH REPORT**

| International application No. |
| --- |
| PCT/JP99/05365 |

**Box I   Observations where certain claims were found unsearchable (Continuation of item 1 of first sheet)**

This international search report has not been established in respect of certain claims under Article 17(2)(a) for the following reasons:

1. ☒   Claims Nos.: 27
   because they relate to subject matter not required to be searched by this Authority, namely:

   The subject matter of claim 27 relates to a method for diagnosis of the human body.

2. ☐   Claims Nos.:
   because they relate to parts of the international application that do not comply with the prescribed requirements to such an extent that no meaningful international search can be carried out, specifically:

3. ☐   Claims Nos.:
   because they are dependent claims and are not drafted in accordance with the second and third sentences of Rule 6.4(a).

**Box II   Observations where unity of invention is lacking (Continuation of item 2 of first sheet)**

This International Searching Authority found multiple inventions in this international application, as follows:

1. ☐   As all required additional search fees were timely paid by the applicant, this international search report covers all searchable claims.

2. ☐   As all searchable claims could be searched without effort justifying an additional fee, this Authority did not invite payment of any additional fee.

3. ☐   As only some of the required additional search fees were timely paid by the applicant, this international search report covers only those claims for which fees were paid, specifically claims Nos.:

4. ☐   No required additional search fees were timely paid by the applicant. Consequently, this international search report is restricted to the invention first mentioned in the claims; it is covered by claims Nos.:

**Remark on Protest**   ☐   The additional search fees were accompanied by the applicant's protest.
☐   No protest accompanied the payment of additional search fees.

Form PCT/ISA/210 (continuation of first sheet (1)) (July 1992)